(19) Europäisches Patentamt
European Patent Office
Office européen des brevets

(11) **EP 3 703 682 B1**

(12) # EUROPEAN PATENT SPECIFICATION

(45) Date of publication and mention of the grant of the patent:
**13.04.2022 Bulletin 2022/15**

(21) Application number: **18804851.6**

(22) Date of filing: **02.11.2018**

(51) International Patent Classification (IPC):
***A61K 31/422*** *(2006.01)* ***A61P 43/00*** *(2006.01)*
***A61P 17/04*** *(2006.01)* ***A61K 31/5517*** *(2006.01)*
***A61K 31/5513*** *(2006.01)* ***A61K 31/55*** *(2006.01)*
***A61K 31/519*** *(2006.01)* ***A61K 31/506*** *(2006.01)*
***A61K 31/4985*** *(2006.01)* ***A61K 31/4545*** *(2006.01)*
***A61K 31/439*** *(2006.01)* ***A61K 31/437*** *(2006.01)*
***A61K 31/42*** *(2006.01)* ***A61K 31/015*** *(2006.01)*
***A61K 31/4184*** *(2006.01)* ***C07D 413/12*** *(2006.01)*
***C07D 471/04*** *(2006.01)* ***C07D 487/04*** *(2006.01)*

(52) Cooperative Patent Classification (CPC):
**C07D 471/04; A61K 31/015; A61K 31/4184; A61K 31/42; A61K 31/422; A61K 31/437; A61K 31/439; A61K 31/4545; A61K 31/4985; A61K 31/506; A61K 31/519; A61K 31/55; A61K 31/5513; A61K 31/5517; A61P 17/04;**
(Cont.)

(86) International application number:
**PCT/US2018/058887**

(87) International publication number:
**WO 2019/090039 (09.05.2019 Gazette 2019/19)**

(54) **COMPOSITIONS AND METHODS FOR THE INHIBITION OF PRURITUS**

ZUSAMMENSETZUNGEN UND VERFAHREN ZUR HEMMUNG VON PRURITUS

COMPOSITIONS ET MÉTHODES POUR L'INHIBITION DU PRURIT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.11.2017 US 201762581420 P**
**07.05.2018 US 201862667843 P**
**14.05.2018 US 201815979105**

(43) Date of publication of application:
**09.09.2020 Bulletin 2020/37**

(73) Proprietor: **The United States of America, as represented by The Secretary, Department of Health and Human Services**
**Bethesda, Maryland 20892-7660 (US)**

(72) Inventors:
- **HOON, Mark A.**
  **Kensington**
  **Maryland 20895 (US)**
- **SOLINSKI, Hans Juergen**
  **32361 Pr. Oldendorf (DE)**
- **INGLESE, James**
  **Bethesda**
  **Maryland 20817 (US)**
- **DRANCHAK, Patricia**
  **Gaithersburg**
  **Maryland 20877 (US)**

(74) Representative: **Maiwald Patent- und Rechtsanwaltsgesellschaft mbH**
**Elisenhof**
**Elisenstraße 3**
**80335 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
**WO-A2-2015/085055**

- **MISHRA SK ET AL: "The cells and circuitry for itch responses in mice.", SCIENCE, vol. 340, no. 6135, 23 May 2013 (2013-05-23), pages 968-971, XP055547118,**
- **MARK A HOON: "Molecular dissection of itch", CURRENT OPINION IN NEUROBIOLOGY., vol. 34, 1 October 2015 (2015-10-01), pages 61-66, XP055546632, GB ISSN: 0959-4388, DOI: 10.1016/j.conb.2015.01.017**
- **DATABASE REGISTRY [Online] CHEMICAL ABSTRACTS SERVICE, COLUMBUS, OHIO, US; 18 September 2012 (2012-09-18), XP002788309, retrieved from STN accession no. 1394592-04-1 Database accession no. 1394592-04-1**

(52) Cooperative Patent Classification (CPC): (Cont.)
**A61P 43/00; C07D 413/12; C07D 487/04**

## Description

STATEMENT REGARDING FEDERALLY SPONSORED RESEARCH AND DEVELOPMENT

This invention was made with Government support under project number DE000721 by the National Institutes of Health, National Institute of Dental and Craniofacial Research. The Government has certain rights in the invention.

BACKGROUND OF THE INVENTION

Pruritus (alternatively spelled, "pruritis"), or itch, is an irritating skin sensation that provokes a desire to scratch. Pruritus may range from mildly unpleasant and temporary to acute and persistent sensations. A number of skin (e.g., fungal infections, and skin conditions such as atopic dermatitis) and systemic conditions (e.g., renal failure, liver damage, liver disease (e.g., cirrhosis), acquired immune deficiency syndrome (AIDS), polycythemia vera, diabetes, hyperthyroidism, and cancer (e.g., Hodgkin's lymphoma, non-Hodgkin's lymphoma, and Kaposi's sarcoma) may be associated with acute and/or chronic pruritus, which can significantly reduce quality of life. Other causes of pruritus may include induction by cytokines or treatments such as chemotherapy and kidney dialysis.

Conventional treatments of pruritus include prescription and over-the-counter medications, including topical anti-inflammatory agents, anti-histamines, and emollients, which are aimed at managing the itchy sensation. However, without eradication of the underlying disease, treatment of pruritus often is ineffective and may be frustrating for patients and prescribing physicians. Thus, the treatment of pruritus continues to be a diagnostic and therapeutic challenge, such that there is a need for improved compositions and methods for treating pruritus.

BRIEF SUMMARY OF THE INVENTION

The invention relates to a pharmaceutical composition comprising a pharmaceutically acceptable carrier and a molecular inhibitor of Natriuretic polypeptide receptor 1 ("Npr1,"), which is a receptor for, *inter alia,* neuropeptide natriuretic polypeptide B ("Nppb" or "NppB"). Details concerning Npr1 and NppB are discussed in U.S. Patent Application No. 15/039,982 (U.S. Patent Application Publication No. 2017-0014486 A1).

The invention provides a pharmaceutical composition comprising (a) a pharmaceutically acceptable carrier and (b) a molecular inhibitor of Natriuretic polypeptide receptor 1 ("Npr1"), wherein the molecular inhibitor of Npr1 is 1-cyclohexyl-3-(cyclopropylmethyl)-N-((3-methylisoxazol-5-yl)methyl)-2-oxo-2,3-dihydro-1H-benzo[d]imidazole-5-carboxamide.

Another embodiment of the invention provides a pharmaceutical composition comprising (a) a pharmaceutically acceptable carrier and (b) a molecular inhibitor of Npr1, for use in the treatment, reduction, or prevention of acute and/or chronic pruritus in a mammal, wherein the molecular inhibitor of Npr1 is 1-cyclohexyl-3-(cyclopropylmethyl)-N-((3-methylisoxazol-5-yl)methyl)-2-oxo-2,3-dihydro-1H-benzo[d]imidazole-5-carboxamide.

Further embodiments of the invention provide the pharmaceutical composition for use in treating, reducing, or preventing acute and/or chronic pruritus in a mammal wherein the acute and/or chronic pruritus is associated with a skin condition, a systemic condition, induced by a pruritogen, or induced by a cytokine. Additional embodiments of the invention provide the pharmaceutical composition for use in treating, reducing, or preventing acute and/or chronic pruritus in a mammal wherein the pruritus is associated with kidney dialysis or chemotherapy.

BRIEF DESCRIPTION OF THE SEVERAL VIEWS OF THE DRAWING(S)

Figure 1A is a schematic depicting the signaling pathway employed in the cell-based assay for assessing Npr1 receptor activity. Npr1 is stimulated by Nppa or Nppb peptides, which elicit activation of cyclase and production of cGMP. In turn, cGMP levels can be measured by cGMP binding to the PDE5 moiety of the cGMP sensor (pGS-40F) through which a conformational change activates *Firefly* luciferase domains producing light.

Figure 1B is a graph showing concentration response curves for HEK293 cells transiently expressing mNpr1 and pGS-40F stimulated by a concentration titration of natriuretic peptides mNppa, mNppb, and Nppc. Increases of light emission in cells stimulated with natriuretic peptides showed that mNpr1 is potently activated by Nppb and Nppa, but is only activated by high concentrations of Nppc. Data represent means ± SEM of duplicate measurements.

Figure 1C is a graph showing HEK293 cells transiently expressing mNpr1 and pGS-40F treated with A-71915. Five minutes after addition of A-71915 (comparative example), cells were treated with 1 nM mNppb. Treatment with mNppb activated cells which is inhibited by A-71915. Data represent means ± SEM of duplicate measurements.

Figure ID is a is a graph showing HEK293 cells transiently expressing mNpr1 and pGS-40F treated with A-71915.

Treatment with A-71915 directly stimulates basal mNpr1 activity showing that it is a partial agonist. Data represent means ± SEM of duplicate measurements.

Figure 1E is a schematic of hNppa and A-71915 showing their similar cyclic structures. Non-proteinogenic amino acids are indicated (Cha, cyclohexylalanine; D-Tic, D-1,2,3,4-tetrahydoisoquinoline-3-carboxylic acid).

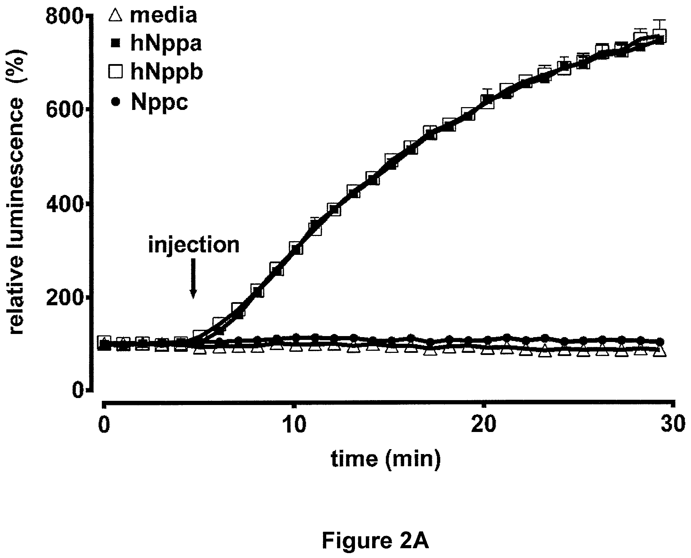

Figure 2A is a graph of a time course experiment showing increases in luciferase activity in stable HEK-hNpr1-cGMP-sensor cells expressing pGS-40F and hNpr1. Natriuretic peptides hNppa and hNppb (10 nM each) elicited increases in luciferase activity. Nppc (10 nM) did not elicit an increase in luciferace activity. Data represent means ± SEM of duplicate measurements.

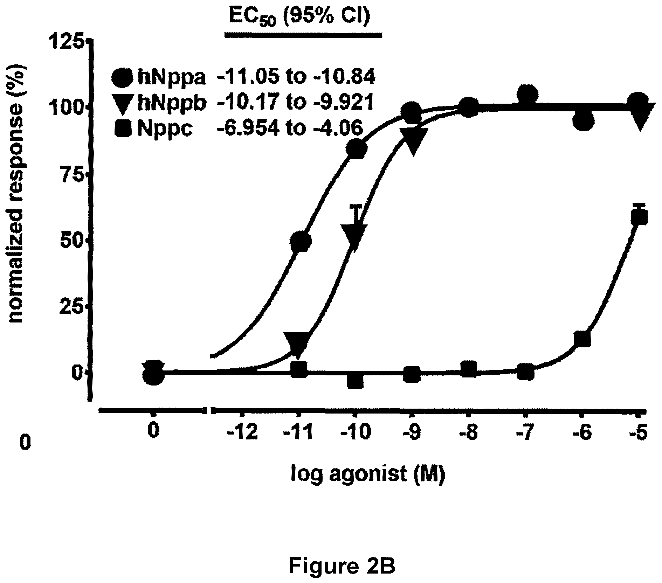

Figure 2B is a graph showing the titration of natriuretic peptides hNppa, hNppb, and Nppc. This demonstrated that HEK-hNpr1-cGMP-sensor cells exhibit the appropriate stimulation potencies to different peptides; rank order of potency of ligands was hNppa > hNppb >>> Nppc. Data represent means ± SEM of duplicate measurements.

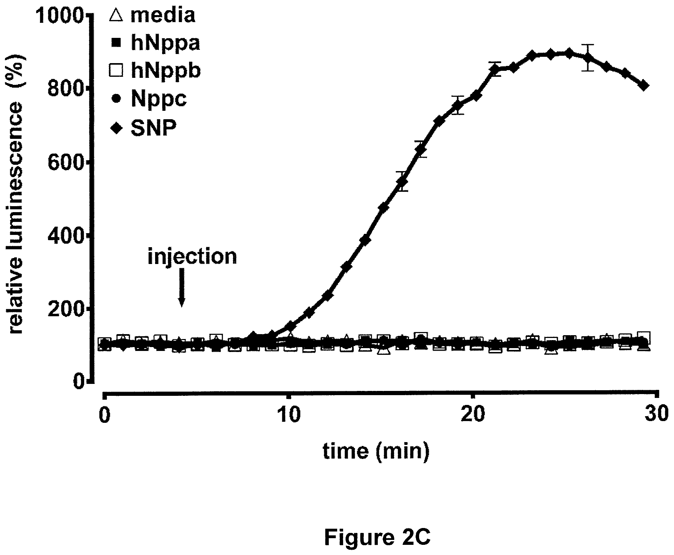

Figure 2C is a graph of a time course experiment showing increases in luciferase activity of stable cells expressing pGS-40F. The HEK-cGMP-sensor cells are stimulated by SNP, but not by natriuretic peptides hNppa, hNppb, and Nppc. Data represent means ± SEM of duplicate measurements.

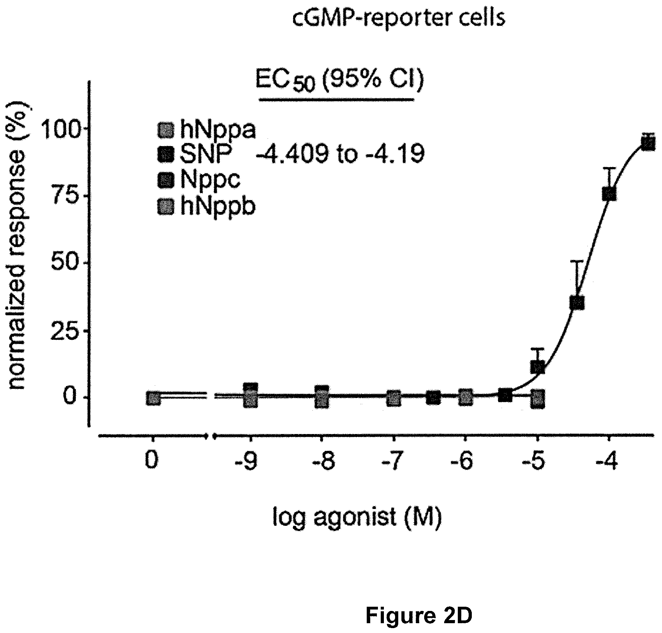

Figure 2D is a graph showing the response in stable HEK-cGMP-sensor cells expressing pGS-40F to hNppa, SNP, Nppc, and Nppb. The HEK-cGMP-sensor cells are stimulated by SNP, but not by natriuretic peptides hNppa,Nppc, or Nppb. Data represent means ± SEM of duplicate measurements.

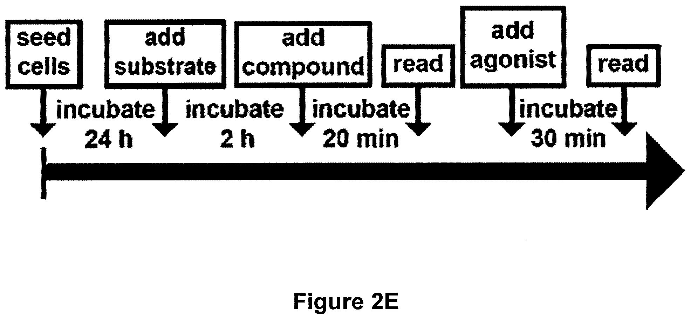

Figure 2E is a schematic depicting the protocol time course for the qHTS assay. Reads before and after agonist addition enabled measurement of effects of compounds on both basal and agonist-induced hNpr1 activity.

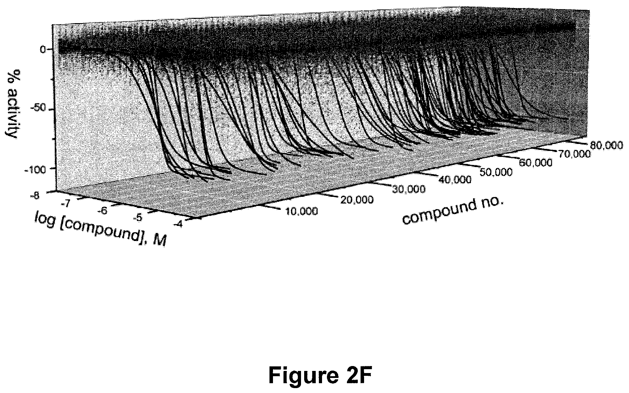

Figure 2F is a 3-axis plot for concentration-response curve profiles for compounds from the Genesis chemical library (86,437 compounds). In total, 519,417 concentration response values are displayed (1574 out-lying values were not plotted). The displayed fit curves (black traces) are for 105 compounds with a maximum antagonism of >90% (i.e., greater than 90%). Curves were fit using a four-parameter logistic regression.

Figure 3A is a graph showing that compound JS-5 (not claimed) inhibits hNppa-induced hNpr1 activity in HEK-hNpr1-cGMP-sensor cells, does not inhibit *Firefly* luciferase (FLuc in *vitro*), does not block SNP-induced GloSensor™ signals (in HEK-cGMP-sensor cells), and does not produce cytotoxicity. Data are measurements from the qHTS assays.

Figure 3B is a schematic diagram of the chemical structure of JS-5.

Figure 3C is a graph showing that compound JS-8 (not claimed) inhibits hNppa-induced hNpr1 activity in HEK-hNpr1-cGMP-sensor cells, does not inhibit *Firefly* luciferase (FLuc in *vitro*), does not block SNP-induced GloSensor™ signals (in HEK-cGMP-sensor cells), and does not produce cytotoxicity. Data are measurements from the qHTS assays.

Figure 3D is a schematic diagram of the chemical structure of JS-8.

Figure 3E is a graph showing that compound JS-11 (according to the claimed invention) inhibits hNppa-induced hNpr1 activity in HEK-hNpr1-cGMP-sensor cells, does not inhibit *Firefly* luciferase (FLuc in *vitro*), does not block SNP-induced GloSensor™ signals (in HEK-cGMP-sensor cells), and does not produce cytotoxicity. Data are measurements from the qHTS assays.

Figure 3F is a schematic diagram of the chemical structure of JS-11.

Figure 4A is a schematic diagram depicting the principle steps of the in *vitro* membrane cyclase assay. A crude membrane fraction is obtained after mechanical disruption of HEK-hNpr1-cGMP-sensor cells (step 1). Incubation of hNpr1 membranes with GTP (step 2) generates cGMP (measured using ELISA) from the natriuretic peptide stimulated receptor and inhibition of this reaction can be assessed (step 3).

Figure 4B is a graph showing that dose dependence of hNpr1 membrane production of cGMP to hNppa and SNP is demonstrative of the specificity and sensitivity of the cell-free assay. Data represent means ± SEM of triplicate (single measurement for SNP).

Figure 4C is a graph showing the inhibition of hNpr1 agonist-induced activity after membranes were incubated with InM hNppa and JS-5, JS-8 or JS-11. Data represent means ± SEM of triplicate and duplicate measurements.

Figure 5A is a graph showing that basal hNpr1 activity (unstimulated activity) in HEK-hNpr1-cGMP-sensor cells was dose dependently inhibited by hNpr1 antagonists JS-5, JS-8, and JS-11. Data represent means ± SEM of triplicate measurements.

Figure 5B is a graph showing the measurement of hNpr1 activity in the cell-based GloSensor™ assay, after hNpr1 expressing cells were treated with a fixed concentration of antagonists (5 μM) prior to the addition of increasing concentrations of hNppa. Data represent means ± SEM of duplicate measurements.

Figure 5C is a graph showing the measurement of hNpr1 activity in the membrane cyclase assay (ELISA-measured), after hNpr1 expressing membranes were treated with a fixed concentration of antagonists (5 μM) prior to the addition of increasing concentrations of hNppa. Data represent means ± SEM of duplicate measurements.

Figure 5D is a graph showing that the inhibition of hNpr1 by JS-8 is reversible. Compared to the control condition (ctrl; cells acutely pre-treated with JS-8 and immediately stimulated with 60 pM hNppa), washed cells did not exhibit inhibition.

Figure 6A is a plot of behavioral testing (rotarod performance) initiated 10 minutes after JS-11 treatment. Motor coordination performance, on an accelerating rod, was not significantly altered by JS-11. Data represent means ± SEM of n = 10 animals.

Figure 6B is a plot of behavioral testing (scratching bouts) initiated 10 minutes after JS-11 treatment. Scratching responses to 100 μg histamine injected intradermally into the nape of the neck were significantly attenuated compared to paired controls by administration of JS-11. Data represent means ± SEM of n = 8 animals.

Figure 6C is a plot of behavioral testing (scratching bouts) initiated 10 minutes after JS-11 treatment. Scratching responses to 8.9 μg CYM5442 injected intradermally into the nape of the neck were significantly attenuated compared to paired controls by administration of JS-11. Data represent means ± SEM of n = 10 animals.

Figure 6D is a photographic image of double-labelling ISH staining of human DRG sections revealing that NPPB and HRH1 and MRGPRX1 (illuminated dots shown in side of white-doted circles) are co-expressed with each other. The expression of Nppb was determined with development with a green amplified agent and Hrh1 with a red agent. Red and green staining was present in the same neurons. DRG sections were counter-stained with DAPI (grey background). Neurons stained for Nppb and itch-receptors are highlighted within the white-dotted profile circles.

Figure 6E is a photographic image of double-labelling ISH staining of human DRG sections revealing that Nppb and MRGPRX1 (illuminated dots shown in side of white-doted circles) are co-expressed with each other. The expression of Nppb was determined with development with a green amplified agent and Mrgprx1 with a red agent. Red and green staining was present in the same neurons. DRG sections were counter-stained with DAPI (grey background). Neurons stained for Nppb and itch-receptors are highlighted within the white-dotted profile circles.

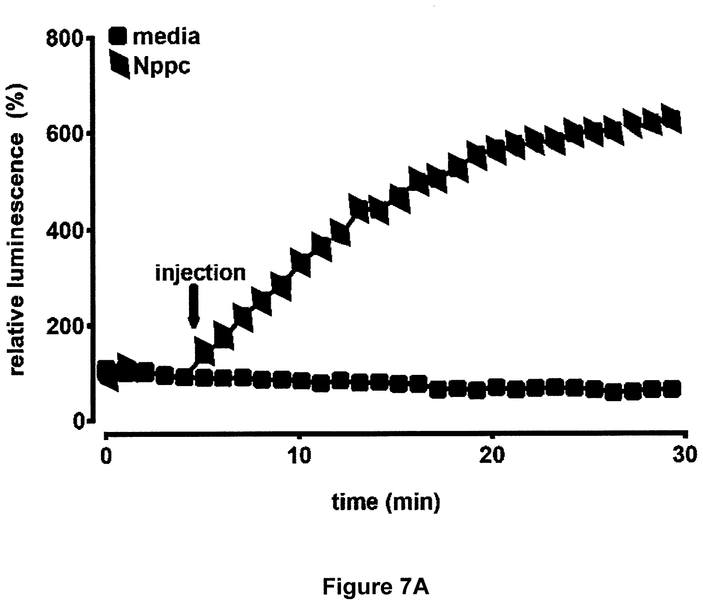

Figure 7A is a graph of time course experiments showing that 10 nM Nppc elicited an increase in luciferase activity in a cloned cell-line stably expressing pGS-40F with hNpr2 (HEK-hNpr2-cGMP-sensor cells). Data represent means ± SEM of duplicate measurements.

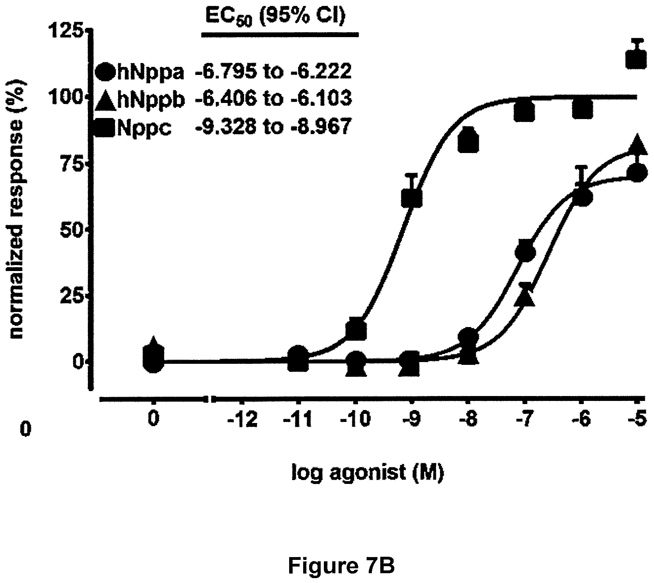

Figure 7B is a graph showing that titration of natriuretic peptides hNppa, hNppb and Nppc in HEK-hNpr2-cGMP-sensor cells exhibited the appropriate stimulation potencies to different peptides; rank order of potency of ligands was Nppc >> hNppa = hNppb. Data represent means ± SEM of duplicate measurements.

Figure 8A is a graph showing that titration of JS-5 antagonizes natriuretic peptide-induced activation of HEK-hNpr1-cGMP-sensor cells (with Nppa) and HEK-hNpr2-cGMP-sensor cells (with Nppc).

Figure 8B is a graph showing that titration of JS-8 antagonizes natriuretic peptide-induced activation of HEK-hNpr1-cGMP-sensor cells (with Nppa) and HEK-hNpr2-cGMP-sensor cells (with Nppc).

Figure 8C is a graph showing that titration of JS-11 antagonizes natriuretic peptide-induced activation of HEK-hNpr1-cGMP-sensor cells (with Nppa) and HEK-hNpr2-cGMP-sensor cells (with Nppc).

Figure 9A is a photographic image of double-labelling ISH staining of human DRG sections revealed that NPPB and IL3 1RA (illuminated dots shown in side of white-doted circles) are co-expressed. The expression of Nppb was determined with development with a green amplified agent and IL3 1RA with a red agent. Red and green staining was present in the same neurons. DRG sections were counter-stained with DAPI (grey background). Neurons stained for Nppb and itch-receptors are highlighted within the white-dotted profile circles.

Figure 9B is a schematic diagram of the experimental procedure for contact hypersensitivity-induced itch, according to embodiments of the invention.

Figure 9C is a plot showing that no differences in % ear thickness were detectable between JS-11 and vehicle-treated control mice. Differences were assessed using paired two-tailed Student's t-test (DMSO: ns P = 0.3965, JS-11: ns P = 0.5401). Data represent means ± SEM of n = 8.

Figure 9D is a plot showing that itch-behavior was significantly reduced by administration of JS-11. Differences were assessed using paired two-tailed Student's t-test (DMSO: ns P = 0.3827, JS-11: *P = 0.0061). Data represent means ± SEM of n = 8.

DETAILED DESCRIPTION OF THE INVENTION

The invention provides a pharmaceutical composition comprising (a) a pharmaceutically acceptable carrier and (b) a molecular inhibitor of Npr1, wherein the molecular inhibitor of Npr1 is 1-cyclohexyl-3-(cyclopropylmethyl)-N-((3-methylisoxazol-5-yl)methyl)-2-oxo-2,3-dihydro-1H-benzo[d]imidazole-5-carboxamide. As used herein, unless stated otherwise, the terms "Nppb" and "Npr1" refer to Nppb and Npr1, respectively, in any form (e.g., mRNA or protein) and from any species (e.g., human or mouse). While, in the context of the invention, the molecular inhibitor can inhibit any mammalian Npr1, it is preferred that it inhibit the human isoform of Npr1, which is referred to herein as "hNpr1."

The pharmaceutically acceptable carrier for use in the inventive pharmaceutical composition can be any of those conventionally used and is limited only by chemico-physical considerations, such as solubility and lack of reactivity with the active compound(s), and by the route of administration. The pharmaceutically acceptable carriers for use in the present invention - for example, vehicles, excipients, and diluents - are well-known to those skilled in the art and are readily available to the public. It is preferred that the pharmaceutically acceptable carrier be one which is chemically inert to the active agent(s) (i.e., the one or more a molecular inhibitor(s) of Npr1) and one which has no detrimental side effects or toxicity under the conditions of use. The choice of carrier will be determined in part by the particular compounds used in the pharmaceutical composition, as well as by the particular method used to administer the one or more molecular inhibitor(s) of Npr1.

The molecular inhibitor of Npr1 for use in the inventive pharmaceutical composition can inhibit the biological activity of Npr1 in any manner, e.g., by inhibiting the production (e.g., expression) of any one or more of Nppb mRNA and Nppb protein, or Npr1 mRNA and Npr1 protein; by inhibiting the binding of Nppb to Npr1 and/or by inhibiting Nppb or Npr1 signaling, as compared to that which is observed in the absence of the molecular inhibitor of Npr1. The biological activity may be inhibited to any degree that realizes a beneficial therapeutic effect. For example, in some embodiments, the biological activity may be completely inhibited (i.e., prevented), while in other embodiments, the biological activity may be partially inhibited (i.e., reduced). The inhibition of hNpr1 is measured by determining the reduced production of cGMP, as described herein with respect to other aspects of the invention.

In an embodiment of the invention, the molecular inhibitor of Npr1 is an agent that inhibits Npr1 signaling. In the context of the present invention, Npr1 signaling can be inhibited in any manner. For example, the molecular inhibitor of Npr1 may inhibit the activation of any one or more of various downstream targets of Nppb or Npr1 signaling. For example, the inhibitor of Npr1 may be an agent that binds to the Nppb protein, thereby reducing or preventing Nppb signaling and inhibiting its function. By way of illustration, the agent that inhibits Npr1 signaling can be a chemical inhibitor, such as, for example, any of the chemical inhibitors described herein. According to the invention, the molecular inhibitor of Npr1 is the molecular inhibitor described herein as compound JS-11, i.e., 1-cyclohexyl-3-(cyclopropylmethyl)-N-((3-methylisoxazol-5-yl)methyl)-2-oxo-2,3-dihydro-1H-benzo[d]imidazole-5-carboxamide.

Molecular inhibitors of Npr1 include small molecules that inhibit Nppb and/or Npr1 signaling, bind the Nppb or Npr1 protein or functional fragment thereof, or compete with the Nppb protein or functional fragment thereof for its native binding site of the Npr1. Suitable inhibitors of Npr1 can include, for example, any one of the chemical compounds described herein.

A molecular inhibitor of Npr1 can be identified using high-throughput screening ("HTS") of chemical compounds. HTS is an automated drug-discovery process that quickly assays the biological or biochemical activity of a large number of chemical compounds (referred to herein as a "library) against a chosen set of defined targets. Screening large numbers of biological modulators across a library of targets produces a number of "active hits," or "actives" which can then be interrogated in detail through secondary hit validation. The actives of interest may be selected as potentially potent modulators for progression to additional, in-depth studies, and compounds with poor or no effect can be dropped from investigation. HTS may be used for discovering ligands for receptors, enzymes, ion-channels, antibodies or other pharmacological targets, or pharmacologically profiling a cellular or biochemical pathway of interest for pharmacological discovery (see e.g., Malo et al., Nat. Biotechnol., 24: 167-175 (2006)). Typically, HTS assays are performed at a single concentration in microtiter plates with a 96, 384, or 1536 well format.

Alternatively, quantitative HTS or "qHTS" can be used to screen for biological modulators such as a molecular inhibitor of Npr1. Quantitative HTS utilizes advanced screening technologies, such as low-volume dispensing, high-sensitivity detectors, and robotic plate handling, to develop a titration-based screening approach (see, e.g., Inglese et al., PNAS USA, 103: 11473-11478 (2006) and Austin et al., Science, 306: 1138-1139 (2004)).

The HTS, qHTS, or other screening technologies typically involve screening a library of many compounds. Such full-scale HTS or qHTS libraries may extend into many thousands of compounds and/or constructs. Libraries are initially divided by composition - e.g. small compound, siRNA, shRNA, etc. Libraries may be further sub-divided into smaller libraries according to biological family or target specificity. Libraries are arrayed into micro-well plates enabling screening in a miniaturized format in 96, 384, and 1536 well plates. An exemplary library of compounds suitable for identifying the molecular inhibitors of Npr1 include the National Institutes of Health National Center for Advancing Translational Sciences ("NCATS") Genesis Chemical Library. This Genesis Chemical Library contains between 80,000 and 100,000 compounds. The collection is plated in qHTS format, enabling large-scale deorphanization of unprecedented mechanisms. The compounds within this Genesis Chemical Library originate from various sources/vendors. Without wishing to be bound, it is believed that most compounds in the Genesis Chemical Library can be purchased directly from the original vendors.

According to the invention, the following compound is used: 1-cyclohexyl-3-(cyclopropylmethyl)-N-((3-methylisoxazol-5-yl)methyl)-2-oxo-2,3-dihydro-1H-benzo[d]imidazole-5-carboxamide (Chembridge Corporation, catalog number 65833004).

The present disclosure discloses a pharmaceutical composition comprising (a) a pharmaceutically acceptable carrier and (b) one or more of the compounds identified as "JS1" through "JS14" in Table 1. Such compounds are present within the aforementioned Genesis Chemical Library and can be obtained commercially from the vendors provided herein. The invention provides a pharmaceutical composition comprising (a) a pharmaceutically acceptable carrier and (b) the compound identified as "JS-11" in Table 1, i.e., 1-cyclohexyl-3-(cyclopropylmethyl)-N-((3-methylisoxazol-5-yl)methyl)-2-oxo-2,3-dihydro-1H-benzo[d]imidazole-5-carboxamide. JS-11 is present within the aforementioned Genesis Chemical Library and can be obtained commercially from the vendor provided herein. In a preferred embodiment, the composition according to the invention is for use in the treatment, reduction, or prevention of acute and/or chronic pruritus in a mammal.

Accordingly, the invention provides a pharmaceutical composition comprising (a) a pharmaceutically acceptable carrier and (b) the molecular inhibitor of Npr1, JS-11, for use in the treatment, reduction, or prevention of acute and/or chronic pruritus in a mammal. Further, the description describes the use of the molecular inhibitor of Npr1, JS-11, for the preparation of a medicament, preferably one suitable for the treatment, reduction, or prevention of acute and/or chronic pruritus in a mammal.

The pruritus may be caused by or associated with any condition or any treatment of a condition. In an embodiment of the invention, the pruritus may be caused by or associated with a skin condition. Examples of skin conditions may include, but are not limited to, skin infections from *Trichomonas* or a fungus, psoriasis, and atopic dermatitis (also known as eczema). In an embodiment of the invention, the pruritus may be caused by or associated with a systemic condition or treatment of a systemic condition. Examples of systemic conditions may include, but are not limited to, renal failure, liver damage, liver disease (e.g., cirrhosis), acquired immune deficiency syndrome (AIDS), polycythemia vera, diabetes, hyperthyroidism, and cancer (e.g., Hodgkin's lymphoma, non-Hodgkin's lymphoma, and Kaposi's sarcoma). Examples of treatments of systemic conditions include, but are not limited to, kidney dialysis and chemotherapy with agents such as, for example, doxorubicin, daunorubicin, cytarabine, paclitaxel, and cisplatin. These chemotherapeutic agents, which are used to treat a variety of cancers, may cause a skin reaction and may be associated with pruritus. The incidence of non-cancer causes of pruritus may depend on the condition and type of treatment.

In an embodiment of the invention, the pruritus is induced by a pruritogen, for example, histamine, chloroquine, endothelin (ET-1), 2-methyl serotonin (5HT), SLIGRL-NH2 (PAR2), and compound 48/80 (48/80).

Also, the pruritus may be induced by a cytokine. In an embodiment of the invention, the pruritus is induced or mediated by interleukin (IL)-31. IL-31 is associated with chronic itch in some types of skin disorders such as, for example, atopic dermatitis.

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the inventive methods can provide any amount of any level of treatment or prevention of pruritus in a mammal. Furthermore, the treatment or prevention provided by the inventive method can include treatment or prevention of one or more conditions or symptoms of the pruritus, e.g., chronic pruritus, being treated or prevented. Also, for purposes herein, "prevention" can encompass delaying the onset of the pruritus, or a symptom or condition thereof. With respect to the inventive methods, the pruritus can be any pruritus, including any of the types of pruritus caused by or associated with any of the conditions or treatments discussed herein.

For purposes of the invention, the amount or dose of the molecular inhibitor of Npr1 administered should be sufficient to effect the desired biological response, e.g., a therapeutic or prophylactic response, in the mammal over a clinically reasonable time frame. The dose will be determined by the efficacy of the particular molecular inhibitor of Npr1 and the condition of the mammal (e.g., human), as well as the body weight of the mammal (e.g., human) to be treated. The dose of the molecular inhibitor of Npr1 also will be determined by the existence, nature and extent of any adverse side effects that might accompany the administration of a particular molecular inhibitor of Npr1.

Typically, the attending physician will decide the dosage of the molecular inhibitor of Npr1 with which to treat each individual patient, taking into consideration a variety of factors, such as age, body weight, general health, diet, sex, molecular inhibitor of Npr1 to be administered, route of administration, and the severity of the condition being treated. The dose, or dosage, of a pharmaceutical composition of the present invention may be appropriately determined by considering the dosage form, method of administration, patient age and body weight, symptoms of the patient, or severity of the condition. Generally, the daily dose for an adult can be, e.g., between 0.1 mg to 10,000 mg at once or in several portions. The dose can be, e.g., 0.2 to 10,000 mg/day (e.g., 1-10 g/day, 2-8 g/day, 1-5 g/day, 0.5 to 2.5 g/day, 0.5 to 500 mg/day, 1 to 300 mg/day, 3 to 100 mg/day, or 5 to 50 mg/day). These doses, or dosages, may vary, depending on the patient body weight and age, and the method of administration; however, selection of suitable dose, or dosage, is well within the purview of those skilled in the art. Similarly, the dosing period may be appropriately determined depending on the therapeutic progress. As used herein, the term "mammal" refers to any mammal, including, but not limited to, mammals of the order Rodentia, such as mice and hamsters, and mammals of the order Logomorpha, such as rabbits. It is preferred that the mammals are from the order Carnivora, including Felines (cats) and Canines (dogs). It is more preferred that the mammals are from the order Artiodactyla, including Bovines (cows) and Swine (pigs) or of the order Perssodactyla, including Equines (horses). It is most preferred that the mammals are of the order Primates, Ceboids, or Simoids (monkeys) or of the order Anthropoids (humans and apes). An especially preferred mammal is the human. The mammal can be non-diseased, a mammal afflicted with pruritus, or a mammal predisposed to pruritus.

In an embodiment of the invention, administering the molecular inhibitor of Npr1 to the mammal may comprise administering the molecular inhibitor of Npr1 orally, intravenously, intramuscularly, subcutaneously, or intraperitoneally. The following formulations for oral, intravenous, intramuscular, subcutaneous, or intraperitoneal administration are exemplary and are in no way limiting. More than one route can be used to administer the molecular inhibitor of Npr1, and in certain instances, a particular route can provide a more immediate and more effective response than another route.

Oral formulations may include any suitable carrier. For example, formulations suitable for oral administration may comprise suitable carriers, such as lactose, sucrose, starch, talc magnesium stearate, crystalline cellulose, methyl cellulose, carboxymethyl cellulose, glycerin, sodium alginate or gum arabic among others.

Intravenous, intramuscular, subcutaneous, or intraperitoneal formulations may include any suitable carrier. For example, formulations suitable for intravenous, intramuscular, subcutaneous, or intraperitoneal administration may comprise sterile aqueous solutions of the molecular inhibitor of Npr1 with solutions which are preferably isotonic with the blood of the recipient. Such formulations may be prepared by dissolving the molecular inhibitor of Npr1 in water or other suitable physiologically acceptable solvent containing physiologically compatible substances such as sodium chloride (e.g. 0.1-2.0M), glycine, and the like, and having a buffered pH compatible with physiological conditions to produce solution (e.g., an aqueous solution or solution of the molecular inhibitor of Npr1 in a suitable physiologically acceptable solvent), and rendering said solution sterile.

The following examples further illustrate the invention but, of course, should not be construed as in any way limiting its scope. In the context of these examples, the term "NppA" refers to natriuretic peptide A, a paralog of NppB, which also acts on the Npr1 receptor. As used herein, the term "NppC" refers to natriuretic peptide C, a second paralog of NppB, which also acts on the Npr1 receptor. Also, the term Npr2 refers to natriuretic peptide receptor 2, the second integral membrane receptor for natriuretic peptides in addition to Npr1, which is discussed herein with respect to other aspects of the invention. Npr2 receptor is related to the Npr1 receptor. Without wishing to be bound, the while Npr1 and Npr2 receptors are related, the agents that stimulate the Npr1 and Npr2 receptors are quite different. For example, for Npr1, NppA is a very strong stimulator (agonist), while NppC is a very weak agonist. The reverse is true for Npr2, i.e., NppC is a strong agonist, while NppA is very weak.

EXAMPLE 1

In order to identify small molecular compounds that inhibit the Npr1 receptor a high-throughput assay to probe inhibition of the receptor guanylate cyclase Npr1 was developed. The assay was designed taking advantage of Promega's GloSensor™ technology (Promega, Madison, WI). This technology involves the expression of a cyclic guanosine monophosphate (cGMP) biosensor, comprised of a firefly luciferase variant that was coupled to the cGMP binding domain of human phosphodiesterase 5A, and loading of cells with a cell-permeable luciferase substrate to monitor cGMP production by Npr1 in real time in live cells. Briefly, human embryonic kidney 293 cells stably expressing the human Npr1 and the pGloSensor™-40F cGMP vector ("pGloSensor™-40F") were generated. Stimulating these cells with the Npr1 agonists human NppA or NppB, luciferase-induced light production could be detected in a dose-dependent manner. HEK293 cells stably expressing human Npr2 and pGS-40F and cells only expressing pGS-40F were also generated to conduct secondary counter screens. In these cells, NppC or sodium nitroprusside were used to stimulate Npr2 or soluble guanylate cyclase which is endogenously expressed in HEK293 cells, respectively. The Npr2-NppC counter screen was employed to search for agents that minimally effected this agonist-receptor interaction. The Npr2-NppC system was used because the side effects of most drugs occur on related receptors. Npr1 and Npr2 are the most related receptors in sequence and function and there are no additional active receptor subtypes in this family of receptors. The procedure is described in detail below.

*Materials*

Nppa, murine Nppa, and A-71915 were purchased from Bachem (Torrance, CA). Human Nppb, CYM5442 and Histamine were purchased from Tocris (Minneapolis, MN), murine Nppb was from Peptide 2.0 (Chantilly, VA), and Nppc was from Mimotopes (Raleigh, NC). JS-3 was purchased from Analyticon Biotechnologies (Lichtenfels, Germany), JS-4 - JS-10 were purchased from Charles River Discovery (Franklin, MA), JS-11 was from Chembridge (San Diego, CA), and JS-12 - JS-14 were from Chemdiv (San Diego, CA). If not indicated otherwise, all other reagents were purchased from ThermoFisher (Waltham, MA) or Sigma-Aldrich (St. Louis, MO).

*In situ Hybridization*

DRGs from 3 different, healthy donors were obtained from the Tissue For Research website and the NIH NeuroBioBank at the University of Maryland (Baltimore, MD). Mouse DRGs were dissected from C57BL/6N mice (Envigo, Indianapolis, IN). ISH staining was performed using the RNAscope® (ACD, Newark, CA) technology according to the manufacturer's instructions. Probes specific for hTRPV1, hNPPB, hHRH1, hMRGPRX1, h1L31RA, mTrpV1, and mNppb in conjunction with the RNAscope® multiplex fluorescent development kit were used and nuclei were counter-stained using DAPI. Since human tissue has high auto-fluorescence, to quench auto-fluorescence after the staining procedure, DRG sections were consecutively treated with True View™ (Vector laboratories, Burlingame, CA) and TrueBlack® (Biotium, Fremont, CA). Images were collected on an Eclipse Ti (Nikon, Melville, NY) confocal laser-scanning microscope using a 40X objective and to enhance visibility of the ISH signals, residual auto-fluorescent signal was background-subtracted with Image J (NIH, Bethesda, MD).

*Eukaryotic Expression Vectors*

The pGloSensor™-40F cGMP vector and coding for a cGMP biosensor, was purchased from Promega (Madison, WI). Expression vectors for murine and human Npr1, MC223390 and SC125506, respectively, were purchased from OriGene Technologies (Rockville, MD). ADDGENE plasmid # 23479 (pDONR223-NPR2) (Cambridge, MA) was obtained as a gift to the laboratory. The sequence coding for human Npr2 was subcloned into the expression vector pCMV6-ENTRY (OriGene Technologies) using standard molecular biology methodology. The generation of pEAK8-GFP is described in Mishra et al., J. Neurosci., 32: 8686-8695 (2012).

*Cell Culture and Transfection*

HEK-293 cells (American Type Culture Collection "ATTC," Manassas, VA) were cultured in DMEM/F12 supplemented with 2 mM L-glutamine, 10% FBS, 100 U/ml penicillin, and 100 μg/ml streptomycin. For transient expression, 8 x $10^5$ cells were seeded in 6-wells, cultured for 24 hours, and then transfected using TransIT®-293 (Mirus, Madison, WI) according to the manufacturer's instructions. After transfection (48 hours), cells were used to measure intracellular cGMP production. HEK-293 cell clones stably expressing pGloSensor™-40F alone (HEK-cGMP-sensor cells) or in addition, human Npr1 ("hNpr1") (HEK-hNpr1-cGMP-sensor cells) or human Npr2 (BEK-hNpr2-cGMP-sensor cells) were generated by co-transfection with pEAK8-GFP and selection with 1 μg/ml puromycin. Stable expression of transgenes was verified by measuring ligand-induced cGMP production with the cGMP biosensor.

*Measurement of Intracellular* cGMP *Production*

Before the measurement (24 hours in advance), 3 x $10^4$ cells were seeded into white 96-well plates (PerkinElmer, Waltham, MA), coated with 0.1% poly(D-lysine), and assays conducted as recommended by the manufacturer (Promega). Medium was aspirated and cells were loaded for two hours at room temperature with 2% GloSensor™ reagent diluted in $CO_2$-independent media (ThermoFisher) supplemented with 2 mM L-glutamine and 10% FBS (assay medium). Luminescence was measured on a BioTek Synergy™ NEO (Winooski, VT) plate reader at room temperature. After a baseline measurement of 3-5 minutes, assay medium as a control or assay medium with agonist was manually added, and luminescence was measured for 30 minutes at ~1/60 Hz. For compounds tested for Npr1 and Npr2 inhibition, the compounds were added after baseline measurements, and luminescence was read for 5 minutes before addition of agonist. Luminescence was then normalized to baseline measurements and plotted against the time. Ligand-induced changes of cGMP production were quantified by determining the area under the curve. To determine efficacy and potency of agonists, ligand-induced changes were normalized to medium only addition and plotted against the ligand concentration. To determine efficacy and potency of agonists, ligand-induced changes were normalized to vehicle addition and plotted against the ligand concentration. To quantify efficacy ($IC_{50}$) and potency ($I_{max}$) of compounds, compound-induced changes of agonist-induced cGMP production were normalized to vehicle controls, plotted against compound concentration, and fit with a four-parameter logistic regression. Effects of A-71915 on basal mNpr1 activity were fitted with a bell-shaped regression (equation indicated below).

*Genesis Library Preparation*

The Genesis library consisted of 86,437 compounds at the time of screening. Samples of all 86,437 library compounds were plated into 384-well plates (Greiner Bio-One, Monroe, North Carolina) comprising a 7-point inter-plate titration with a serial dilution of 1:5 in DMSO ranging from 10 mM to 640 μM. Plates were reformatted in quadrants into 1536-well format using an EVOLUTION P3 System (PerkinElmer).

*Quantitative HTS*

Measurement of intracellular cGMP production in HEK-hNpr1-cGMP-sensor cells was scaled to 4 μl volumes in 1536-well format and primary screening was performed in an automated manner in 7- to 11-point qHTS. Cells (1.5 x $10^3$) were plated in assay medium in white, solid bottom TC treated 1536-well plates (Greiner Bio-One) with a Multidrop™

Combi Reagent Dispenser (Thermo Fisher Scientific). Cells were incubated at 37 °C, 5% $CO_2$ and 95% humidity for 16 hours and 1 μl of GloSensor™ reagent at a final assay concentration of 2% or assay medium as background control was added to respective wells with a BioRAPTR Flying Reagent Dispenser™ (Beckman Coulter, Schaumburg, IL). Cells were incubated at room temperature for 2 hours, protected from light. Compound (23 nl) was transferred by a pintool (Kalypsys, San Diego, CA) in the concentration range of 3.0 pM to 46.1 μM along with DMSO and a titration of A-71915 from top concentration 38.3 μM to 1.7 nM in columns 1-4 of each plate as vehicle and positive controls, respectively. Cells were incubated with compound for 30 minutes at room temperature, protected from light. Pre-agonist luminescence for each plate was read on a ViewLux™ plate reader (PerkinElmer). Human NppA (1 μl) at a final assay concentration of 0.1 nM, or $CO_2$-independent media were added to respective wells with a BioRAPTR Flying Reagent Dispenser™ (Beckman Coulter). Plates were incubated for 20 minutes at room temperature, protected from light, and luminescence was read on a ViewLux™ plate reader.

Pre-agonist and post-agonist data were normalized by plate to corresponding intra-plate controls as described in Inglese et al., PNAS USA, 103: 11473-11478 (2006). Assay statistics including S:N and Z' were calculated using the same respective controls. Pre-agonist data were normalized to the average signal of DMSO-treated wells with 2% GloSensor™ reagent as 0% and the average signal of DMSO-treated wells with assay medium as -100% activity. Post-agonist data were normalized to the average signal of DMSO-treated wells with 2% GloSensor™ reagent and 0.1 nM hNppA as 0% and the average signal of wells treated with 38.3 μM A-71915 with 2% GloSensor™ reagent and 0.1 nM hNppA as -100% activity. The respective normalized data from each assay plate was corrected using DMSO only treated assay plates at the beginning and end of the screen and interspersed every 20-30 plates throughout screening. In-house software was used to fit the resulting inter-plate titration data to the standard hill equation and concentration-response curves were classified by activity. Curve fit assignments of 1.1, 1.2, 2.1, and 2.2 were considered active and visually confirmed. These data were refit in GraphPad Prism™ 7.0 (GraphPad Software, Inc., La Jolla, California) with nonlinear regression log(agonist) vs. response - variable slope (four parameters) fit. Bell-shaped curves were fit in Graphpad Prism™ 7.0 (GraphPad) with nonlinear regression and the equation:

$$Y = S0 + \frac{(S1 - S0)}{(1 + 10^{((\log EC50 - X)*HillSlope\ 1)})} + \frac{(S2 - S1)}{(1 + 10^{((\log IC50 - X)*HillSlope\ 2)})}$$

*qHTS Follow-Up of Actives*

Active molecules were prioritized based on post-agonist concentration-response curves, maximum efficacy and potency with minimal activity in the corresponding pre-agonist data. The compounds selected for follow-up, 1,408 compounds in total, were replated in 11-pt inter-plate titration with a serial dilution of 1:3 in DMSO ranging from 10 mM to 169 μM. The primary screening assay was repeated with the follow-up compounds as described above. Four different counter screens were conducted to eliminate false positive compounds.

To account for compounds selected because of their inhibition in qHTS, but which selection may have been dependent on interference with components of the assay rather than direct inhibition of hNpr1, several overlapping strategies were used to identify *bona fide* hNpr1 inhibitors and eliminate false positives, including conducting the four different counter screens described below. First, hNpr1 assays were repeated, at 11 concentrations on all selected compounds, to validate all positive compounds from the primary screen. This confirmed the selection criteria used for the selection of the active compounds. Compounds which interfered directly with luciferase, or molecules which are cytotoxic were subtracted using two different assays as described in Jang, et al. "Identification of drug modulators targeting gene-dosage disease CMT1A," ACS Chem Biol 7, 1205-1213 (2012). Third, to eliminate compounds that directly blocked cGMP sensor activity, or sequestered the GloSensor™ reagent, the selected compounds were tested for the inhibition of luciferase activity upon activating soluble guanylate cyclases in HEK293-cGMP sensor cells (using sodium nitroprusside "SNP"). The counter-screens eliminated most compounds with only 20 candidates remaining after all screens were completed. Characteristics of fourteen of these candidates are provided in the tables below.

*HEK293-hNpr2-cGMP-Sensor and HEK293-cGMP-Sensor Cells Counter Screens*

Counter-screens were performed with HEK293-hNpr2-cGMP-sensor and HEK293-cGMP-sensor cells. These cells were stimulated with 1 nM NppC or 50 μM sodium nitroprusside, respectively, but otherwise the assay was conducted as described for HEK-hNpr1-cGMP-sensor cells. Pre-agonist controls were the same for all three assays, but normalization of post-agonist data differed between assays. For HEK-hNpr1-cGMP-sensor cells, -100% activity in post-agonist data was defined as described for primary screening, while for HEK-hNpr2-cGMP-sensor and HEK-cGMP-sensor cells, the average signal of DMSO-treated wells with 2% GloSensor™ reagent and the respective agonist was defined as 0% and the average signal of wells without agonist as -100% activity and data were normalized accordingly. Compounds directly interacting with the pGloSensor™-40F reporter system in HEK293 cells stimulated with sodium nitroprusside were eliminated as false positives. Effects of compounds on HEK293 cells stimulated with NppC and stably expressing hNpr2 and pGS-40F were tested for receptor selectivity.

*Cytotoxicity Counter-Screen*

Compound cytotoxicity was tested with Promega's CellTiter-Glo™ assay which measures cellular adenosine triphosphate content, a surrogate of metabolic activity, with a firefly luciferase reagent. A cytotoxicity counter-screen using HEK-hNpr1-cGMP-sensor cells was performed on the 1,408 follow-up compounds. Cells (1.5 x $10^3$) cells were plated in 4 μL $CO_2$-independent media in white, solid bottom TC-treated 1536-well plates (Greiner Bio-One). Cells were incubated at 37 °C, 5% $CO_2$ and 95% humidity for 16 hours. Compound transfer was performed as described for qHTS along with DMSO and a titration of Digitonin from a top concentration of 115 μM to 3.5 nM in columns 1-4 of each assay plate as vehicle and cytotoxicity controls, respectively. Cells were incubated with compound for 2 hours at 37 °C. CellTiter-Glo™ reagent (Promega) (3 μl) was added to each well with a BioRAPTR Flying Reagent Dispenser™ (Beckman Coulter) and plates were incubated for 10 min at room temperature, protected from light. Luminescence was measured on a ViewLux™ plate reader. Data were normalized to the average signal of DMSO-treated wells as 0% and the average signal of wells treated with 115 μM Digitonin as -100% activity and analyzed as above.

*Biochemical Firefly Luciferase Enzymatic Counter-Screen*

Direct effects of compounds on firefly luciferase enzymatic activity were tested *in vitro.* A Firefly luciferase enzyme assay was performed on the 1,408 follow-up compounds. To achieve half-maximal reaction velocity, the D-Luciferin substrate concentration equaled Km and the assay was otherwise performed according to Jang et al., ACS Chem. Bio., 7: 1205-1213 (2012), as follows: 3 μl of substrate-buffer solution (0.01 mM D-Luciferin, 0.01 mM ATP, 50 mM Tris Acetate, 10 mM Mg Acetate, 0.01% Tween-20 and 0.038% BSA final concentrations) were dispensed into white, solid bottom medium bind 1536-well plates (Greiner Bio-One) with a BioRAPTR Flying Reagent Dispenser™ (Beckman Coulter). Compounds were transferred as described for qHTS along with DMSO (as a negative control) and titrations of PTC124 (as a positive control), ranging from the top concentration of 57.5 μM to 4.0 pM in columns 1-4 of each plate as vehicle and positive controls, respectively. Luciferase enzyme-buffer solution (1 μl) (10 nM *P. pyralis* luciferase in 50 mM Tris Acetate final concentration) was dispensed and after a 5-minute incubation at room temperature, luminescence was read on a ViewLux™ plate reader. Data were normalized to the average signal of DMSO-treated wells as 0% (which indicates that the tested compound does not inhibit luciferase in the assay) and the average signal of wells treated with 57.5 μM PTC124 as -100% activity (which indicates that the tested compound is inhibiting luciferase in the assay) and processed as above.

*Membrane Fractionation*

Two days before membrane fractionation, 8 x $10^6$ HEK-hNpr1-cGMP-sensor cells or HEK-hNpr2-cGMP-sensor cells were seeded in 150 mm dishes to grow to confluence. Medium was aspirated and cells were washed with ice-cold PBS without $Ca^{2+}$/$Mg^{2+}$. Cells were scraped loose in ice-cold 2.5 ml homogenization buffer (HEPES 50 mM, EDTA 5 mM, NaCl 50 mM, NaF 50 mM, microcystin 250 nM, glycerol 20%, cOmplete™ Ultra Mini protease inhibitor cocktail) with a rubber policeman and lysed with a glass-Teflon homogenizer on ice. To remove debris, the lysate was spun for 10 minutes at 500 x g and 4 °C. While the supernatant was stored on ice, the pellet was lysed again with a glass-Teflon homogenizer and centrifuged again for 10 minutes at 500 x g and 4 °C. Both supernatants were pooled and spun for 60 minutes at 100,000 x g and 4 °C in an Optima™ XL-90 Ultracentrifuge (Beckman Coulter) equipped with a 70-Ti rotor. The supernatant was discarded and the pellet was resuspended in membrane buffer (HEPES 10mM, EDTA 1 mM, microcystin 250 nM, cOmplete™ Ultra Mini protease inhibitor cocktail) by passing it through a 25ga needle multiple times. Protein content in these membrane fractions was assessed by a Pierce™ BCA Protein assay kit (ThermoFisher) according to the manufacturer's instructions. Membranes were aliquoted to 100 μg protein, flash frozen in liquid nitrogen, and stored at -80 °C.

*Measurement* of *Npr1 Enzymatic Activity in Membrane Fractions*

For measurement of Npr1 cyclase activity, per single reaction, 2 μg of membranes were diluted to 20 μl with $H_2O$ and mixed with 2.5 μl compound to incubate for 5 minutes. Then, the enzymatic reaction was started by adding pre-warmed 25 μl 2-fold concentrated assay buffer (HEPES 25 mM, EDTA 1 mM, ATP 1mM, $MgCl_2$ 5 mM, microcystin 500 μM, GTP 2 mM final concentrations) with and without Npr1/2 agonist and allowed to proceed for 5 minutes at 37 °C. The reaction was stopped by addition of 19.3 μl of 1 M hydrochloric acid and mixing on a shaker for 10 minutes.

Samples were diluted appropriately and analyzed for cGMP content using a competitive colorimetric immunoassay (ADI-901-014, Enzo Life Sciences, Farmingdale, NY) according to the manufacturer's instructions. To account for differences in cGMP content of starting material, a mock reaction that lacked the substrate GTP was performed and subtracted from experimental samples. To quantify efficacy ($IC_{50}$) and potency ($I_{max}$) of compounds, compound-induced changes of agonist-induced cGMP production were normalized to vehicle controls, plotted against the compound concentration, and data was fit with a four-parameter logistic regression.

*Kinetic Solubility Assay*

The μSOL assay was used for kinetic solubility determination, as described in Avdeef, et al., "Drug absorption in vitro model: filter-immobilized artificial membranes. 2. Studies of the permeability properties of lactones in Piper methysticum Forst," Eur J Pharm Sci 14, 271-280 (2001). In this assay, the classical saturation shake-flask solubility method was adapted as described. Test compounds were prepared as 10 mM DMSO stock solutions and diluted to a final compound concentration of 150 μM in aqueous solution (pH 7.4, 100 mM phosphate buffer). Samples were incubated at room temperature for 6 hours and vacuum-filtered using Te-Vac (Tecan, Morrisville, NC) to remove any precipitates. The concentration of the compound in the filtrate was measured via UV absorbance (λ: 250-498 nm). The unknown compound concentration was determined by comparing the fully solubilized reference plate which contained 17 μM of compound dissolved in spectroscopically pure n-propanol. All compounds were tested in duplicates. The kinetic solubility (μg/mL) of compounds was calculated using the μSOL Evolution software (Pion Inc., Billerica, MA). The three controls used were albendazole (low solubility), phenazolpyridine (moderate solubility) and furosemide (high solubility).

*Rat Liver Microsome Stability Assay*

Single time point microsomal stability was determined in a 96-well HTS format. Sample preparation was automated using an EVO 200 robot (Tecan, Switzerland). A high-resolution LC/MS (Thermo QExactive, ThermoFisher (Waltham, MA)) instrument was used to measure the percentage of compound remaining after incubation using a known method. Six standard controls were tested in each run: buspirone and propranolol (for short half-life), loperamide and diclofenac (for short to medium half-life), and carbamazepine and antipyrine (for long half-life). Briefly, the incubation consisted of 0.5 mg/mL microsomal protein, 1.0 μM compound concentration, and NADPH regeneration system (containing 0.650 mM NADP+, 1.65 mM glucose 6-phosphate, 1.65 mM $MgCl_2$, and 0.2 U/mL G6PDH) in 100 mM phosphate buffer at pH 7.4. The incubation was carried out at 37 °C for 15 min. The reaction was quenched by adding 555 μL of acetonitrile (~1:2 ratio) containing 0.28 μM albendazole (internal standard).

*Parallel Artificial Membrane Permeability Assay (PAMPA)*

Stirring double-sink PAMPA method was employed to determine the permeability of compounds via PAMPA. The PAMPA lipid membrane consisted of an artificial membrane of a proprietary lipid mixture and dodecane (Pion Inc., Billerica, MA), optimized to predict gastrointestinal tract passive permeability. The lipid was immobilized on a plastic matrix of a 96-well "donor" filter plate placed above a 96-well "acceptor" plate. pH 7.4 solution was used in both donor and acceptor wells. The test articles, stocked in 10 mM DMSO solutions, were diluted to 0.05 mM in aqueous buffer (pH 7.4), and the concentration of DMSO was 0.5% in the final solution. During the 30-minute permeation period at room temperature, the test samples in the donor compartment were stirred using the Gutbox technology (Pion Inc.) to reduce the aqueous boundary layer. The test article concentrations in the donor and acceptor compartments were measured using a UV plate reader (Nano Quant, Infinite® 200 PRO, Tecan). Permeability calculations were performed using Pion Inc. software and were expressed in units of 10-6 cm/s. Compounds with low or weak UV signal were analyzed using high resolution LC/MS (Thermo QExactive, Thermo Fisher).

The potency of inhibition in both GloSensor™ and biochemical assays as well as the chemical structure of each compound are summarized in Table 1.

**Table 1.**

| Abr name | IUPAC name | SMILES | $IC_{50}$ GloSensor™ assay (M) | $IC_{50}$ biochem assay (M) |
|---|---|---|---|---|
| JS1* | 4-(cyclohexyl methyl)-4,5-dihydrospiro[benzo[e][1,4]diazepine-3,4'-piperidin]-2(1H)-one hydrochloride | Cl.O=C1NC2=CC=CC=C2CN(CC3CCCCC3)C14CCNCC4 | 7.31E-06 | not determined |

(continued)

| Abr name | IUPAC name | SMILES | $IC_{50}$ GloSensor™ assay (M) | $IC_{50}$ biochem assay (M) |
|---|---|---|---|---|
| JS2* | 2-(4-chlorophenyl)-1-(1'-(pyrrolidin-3-ylmethyl)-1,5-dihydrospiro[benzo[c]azepine-4,2'-pyrrolidin]-2(3H)-yl)ethan-1-one | ClC1=CC=C(CC(=O)N2CC3=CC=CC=C3CC4(CCCN4CC5CCNC5)C2)C=C1 | 1.30E-05 | not determined |
| JS3* | N-(((2R,4S,5R)-5-(6-(penta n-3-yl)-2-(pyridin-4-yl)pyrimidin-4-yl)quinuclidin-2-yl)methyl)thiophene-2-sulfonamide | CCC(CC)C1=NC(=NC(=C1)[C@ H]2CN3CC[C@H]2C[C@@H]3 CN[S+]([O-])(=O)C4=CC=CS4)C5=CC=NC= C5 | 1.41E-05 | 3.98E-06 |
| JS4* | N-(1-(3-methoxy-5-(pyridin-4-yl)benzyl)-4-methylpiperidin-4-yl)acetamide | COC1=CC(=CC(CN 2CCC(C)(CC2 )NC(C)=O)=C1)C3=CC=NC=C3 | 1.41E-05 | 2.20E-06 |
| JS5* | 3,5-dimethyl-4-(6-(2-phenoxyphenyl)imidazo[1,5-a]pyridin-8-yl)isoxazole | CC1=C(C(C)=NO1)C2=CC(=CN3C=NC=C23)C4=CC=CC=C4OC5=CC=CC=C5 | 2.82E-05 | 1.41E-07 |
| JS6* | 3-(2-methoxyphenyl)-N-(pyridin-3-ylmethyl)-1H-pyrazolo [3,4-b]pyrazine-5-carboxamide | COC1=CC=CC=C1C2=NNC3=C2N=C(C=N3)C(=O)NCC4=CC=CN=C4 | 1.12E-05 | 7.66E-07 |
| JS7* | 3-phenyl-N-(pyridin-3-ylmethyl)-1H-pyrazolo[3,4-b]pyrazine-5-carboxamide | O=C(NCC1=CC=CN=C1)C2=NC3=C(NN=C3C4=CC=CC=C4)N= C2 | 2.24E-05 | 1.22E-07 |
| JS8* | 2-(benzo[d] [1,3]dioxol-5-yl)-1-(3-(2-(benzyloxy)phenyl)-2-methyl-6,7-dihydropyrazolo[1,5-a]pyrimidin-4(5H)-yl)ethan-1-one | CC1=NN2CCCN(C(=O)CC3=CC=C4OCOC4=C3)C2=C1C5=CC=CC=C5OCC6=CC=CC=C6 | 8.91E-06 | 3.36E-07 |
| JS9* | 1-(3-(2-(benzyloxy)phenyl)-2-methyl-6,7-dihydropyrazolo[1,5-a]pyrimidin-4(5H)-yl)-2-(pyridin-2-yl)ethan-1-one | CC1=NN2CCCN(C(=O)CC3=CC=CC=N3)C2=C1C4=CC=CC=C4OCC5=CC=CC=C5 | 8.91E-06 | 2.17E-06 |
| JS10* | 2-(benzo[d][1,3]dioxol-5-yl)-1-(2-methyl-3-(2-phenoxyphenyl)-6,7-dihydropyrazolo[1,5-a]pyrimidin-4(5H)-yl)ethan-1-one | CC1=NN2CCCN(C(=O)CC3=CC=C4OCOC4=C3)C2=C1C5=CC=CC=C5OC6=CC=CC=C6 | 7.94E-06 | 7.44E-07 |
| JS11** | 1-cyclohexyl-3-(cyclopropylmethyl)-N-((3-methylisoxazol-5-yl)methyl)-2-oxo-2,3-dihydro-1H-benzo[d]imidazole-5-carboxamide | CC1=NOC(CNC(=O)C2=CC=C3 N(C4CCCCC4)C(=O)N(CC5CC5)C3=C2)=C1 | 5.62E-06 | 1.14E-06 |
| JS12* | (1-(4-methoxyphenyl)cyclopropyl)(5-methyl-5,6-dihydrospiro[benzo[f]imidazo[1,2-a][1,4]diazepine-4,4'-piperidin]-1'-yl)methanone | COC1=CC=C( C=C1 )C2( CC2)C( =O)N3CCC4(CC3)N(C)CC5=C(C=CC=C5)N6C=CN=C46 | 8.91E-06 | 6.14E-07 |
| JS13* | N-benzyl-1-(2-cyanobenzyl)-4-oxo-1,5-diazacycloundecane-8-carboxamide | O=C(NCC1=CC=CC=C1)C2CCC N(CC3=C(C=CC=C3)C#N)CCC(= O)NCC2 | 3.98E-06 | 1.19E-06 |

(continued)

| Abr name | IUPAC name | SMILES | $IC_{50}$ GloSensor™ assay (M) | $IC_{50}$ biochem assay (M) |
|---|---|---|---|---|
| JS14* | N-benzyl-N-methyl-8-phenylimidazo[1,2-a]pyrazine-2-carboxamide | C1(N=C(N(C=1)C=C1)C(=N1)C( =CC1)C=CC=1)C(N(CC(=CC1)C =CC=1)C)=O | 6.31E-06 | 1.86E-05 |
| * not claimed<br>** according to the claimed invention | | | | |

*Data Analysis*

GraphPad Prism™ 7.0 (GraphPad) was used to fit concentration-response curves and calculate $IC_{50}$ and $I_{max}$ values. Differences between mean values were analyzed using two-tailed Student's t-test (two groups) or one-way analysis of variances (ANOVA) with Tukey's post-hoc test (more than two groups). Differences were considered significant for p<0.05 *, p<0.01 **, and p<0.001 ***.

The counter-screens that were performed to eliminate false positives from among the candidate inhibitors led to the identification of a small group of about 20 hNpr1 antagonists, of which fifteen are described in the tables herein. These fifteen compounds also inhibited hNpr2 but had no direct effect on *Firefly* luciferase, were not cytotoxic, and did not interfere with SNP-induced GloSensor™ signals. The fifteen most efficacious hNpr1 inhibitors from the GloSensor™ assays were selected for the validation assay and inhibitory effects were corroborated for each compound. In particular, the apparent potency ($IC_{50}$) and efficacy ($I_{max}$) in five different assays for each candidate compound was tabulated: Inhibition of hNppa-stimulated HEK-hNpr1-cGMP-sensor cells, inhibition of Nppc-stimulated HEK-hNpr2-cGMP-sensor cells, inhibition of SNP-stimulated HEK-cGMP-sensor cells, inhibition of Firefly luciferase in vitro, and cytotoxicity on HEK-hNpr1-cGMP-sensor cells. Concentration-response-curve in each assay was fit with a four-parameter logistic regression. For counter-screens, potency and efficacy of compounds frequently could not be determined as fits did not converge (n. c.) and inhibition was not detectable at all (n. d.), respectively. For each compound, potency (as the $IC_{50}$ in $\mu$M) and efficacy (as $I_{max}$ in %), are given in Table 2.

**Table 2.** qHTS and Various Counter-screens Reveal *bona-fide* hNpr1 Inhibitors.

| Compound | HEK-hNpr1-cGMP-sensor cells | | HEK-hNpr2-cGMP-sensor cells | | HEK-cGMP-sensor cells | | Fluc *in vitro* | | cytotoxicity | |
|---|---|---|---|---|---|---|---|---|---|---|
| | $IC_{50}$ | $I_{max}$ | $IC_{50}$ | $I_{max}$ | $IC_{50}$ | $I_{max}$ | $IC_{50}$ | $I_{max}$ | $IC_{50}$ | $I_{max}$ |
| JS-1* | 26 ± 1.5 | -119 ± 20 | 7.2 ± 1.3 | -106 ± 8 | n. c. | n. d. | 0.03 ± 1.5 | -3 ± 3 | n. c. | n. d. |
| JS-2* | 25 ± 1.3 | -102 ± 9 | 6.4 ± 1.1 | -88 ± 5 | n. c. | n. d. | n. c. | n. d. | n. c. | n. d. |
| JS-3* | 62 ± 19 | -183 ± 67 | 17 ± 20 | -126 ± 23 | n. c. | n. d. | 25 ± 4 | -1 ± 6 | n. c. | n. d. |
| JS-4* | 16 ± 13 | -110 ± 10 | 11 ± 1.2 | -100 ± 5 | n. c. | n. d. | n. c. | n. d. | n. c. | n. d. |
| JS-5* | 32 ± 13 | -127 ± 12 | 12 ± 1.1 | -97 ± 4 | n. c. | n. d. | n. c. | n. d. | n. c. | n. d. |
| JS-6* | 24 ± 1.2 | -15 ± 3 | 6.9 ± 1.1 | -102 ± 3 | n. c. | n. d. | n. c. | n. d. | 45 ± 43 | -36 ± 87 |
| JS-7* | 85 ± 22 | -216 ± 97 | 16 ± 1.3 | -122 ± 11 | n. c. | n. d. | n. c. | n. d. | n. c. | n. d. |
| JS-8* | 13 ± 1.2 | -122 ± 9 | 9.1 ± 1.4 | -127 ± 13 | n. c. | n. d. | n. c. | n. d. | 49 ± 14 | -22 ± 29 |
| JS-9* | 35 ± 15 | -106 ± 18 | 20 ± 16 | -102 ± 16 | n. c. | n. d. | n. c. | n. d. | n. c. | n. d. |
| JS-10* | 17 ± 1.3 | -122 ± 12 | 5.4 ± 1.4 | -86 ± 5 | n. c. | n. d. | n. c. | n. d. | n. c. | n. d. |
| JS-11** | 21 ± 1.3 | -140 ± 13 | 5.0 ± 1.1 | -97 ± 4 | n. c. | n. d. | n. c. | n. d. | n. c. | n. d. |
| JS-12* | 16 ± 1.3 | -116 ± 11 | 5.7 ± 1.1 | -96 ± 4 | n. c. | n. d. | n. c. | n. d. | n. c. | n. d. |
| JS-13* | 10 ± 1.2 | -99 ± 6 | 5.9 ± 1.3 | -108 ± 8 | n. c. | n. d. | n. c. | n. d. | n. c. | n. d. |
| JS-14* | 117 ± 75 | -286 ± 966 | 20 ± 1.1 | -91 ± 8 | n. c. | n. d. | n. c. | n. d. | n. c. | n. d. |
| JS-15* | 6.9 ± 2.0 | -41 ± 8 | 41 ± 13.8 | -41 ± 8 | n. c. | n. d. | n. c. | n. d. | n. c. | n. c. |

* not claimed

** according to the claimed invention

Additional screens of the fifteen candidates corroborated hNpr1 inhibition. In particular, HEK-hNpr1-cGMP-sensor cells were seeded in 96-wells and stimulated with 60 pM hNppa 5 minutes after addition of candidate compounds or A-71915 and luminescence was measured for 30 minutes. Inhibitors were titrated to calculate apparent potency ($IC_{50}$) and efficacy ($I_{max}$) of inhibition of hNppa-induced cGMP production. The results are set forth in Table 3.

Table 3. Detailed Analysis of Candidate Compounds in GloSensor™ assay, Conducted in 96-well Format, Corroborates hNpr1 Inhibition.

| Compound | $IC_{50}$ [μM] (mean ± SEM) | Imax [%] (mean ± SEM) |
|---|---|---|
| JS-3* | 3.1 ± 0.6 | -99.4 ± 0.6 |
| JS-4* | 1.1 ± 0.01 | -95.5 ± 1.2 |
| JS-5* | 7.4 ± 0.3 | -99.9 ± 0.1 |
| JS-6* | 1.9 ± 0.1 | -94.2 ± 5.3 |
| JS-7* | 1.2 ± 0.5 | not determined |
| JS-8* | 1.2 ± 0.1 | -98.8 ± 0.7 |
| JS-9* | 3.1 ± 1.4 | -93.4 ± 64 |
| JS-10* | 6.7 ± 6.7 | -98.3 ± 1.7 |
| JS-11** | 1.9 ± 0.8 | -96.3 ± 1.3 |
| JS-12* | 1.3 ± 0.002 | -96.0 ± 1.3 |
| JS-13* | 1.1 ± 0.1 | -96.1 ± 0.4 |
| JS-14* | 3.1 ± 1.1 | not determined |
| A-71915*** | 1.1 ± 0.1 | -99.8 ± 0.1 |

* not claimed
** according to the claimed invention
*** comparative example

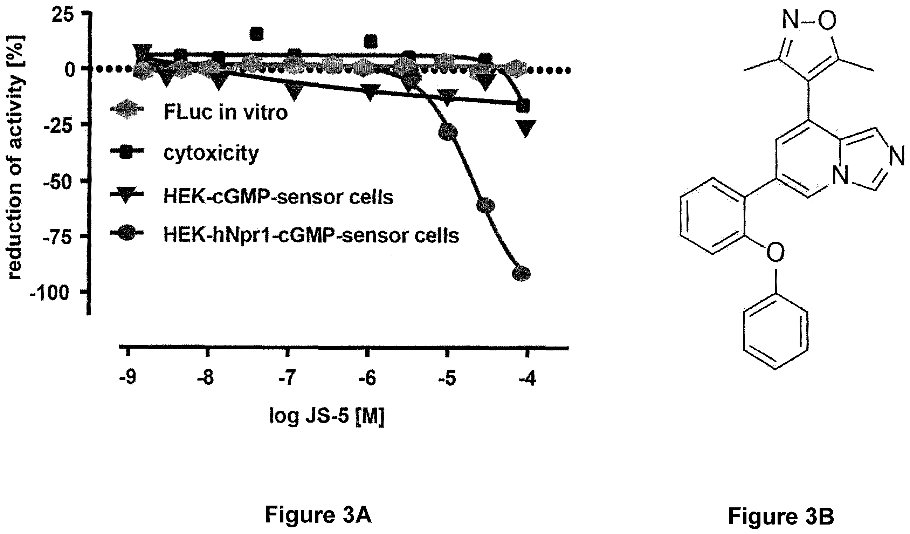

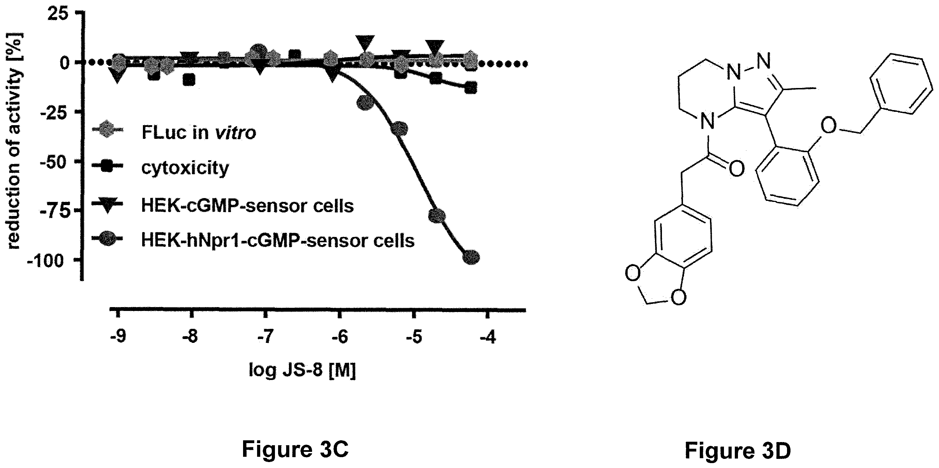

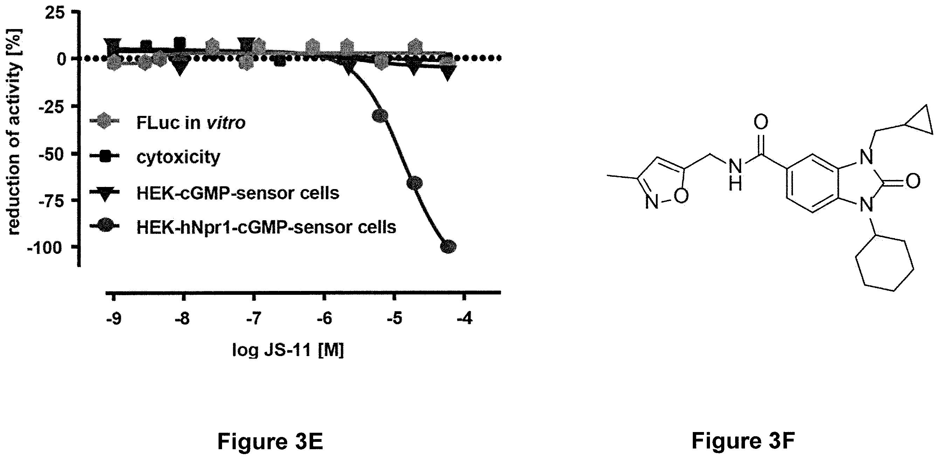

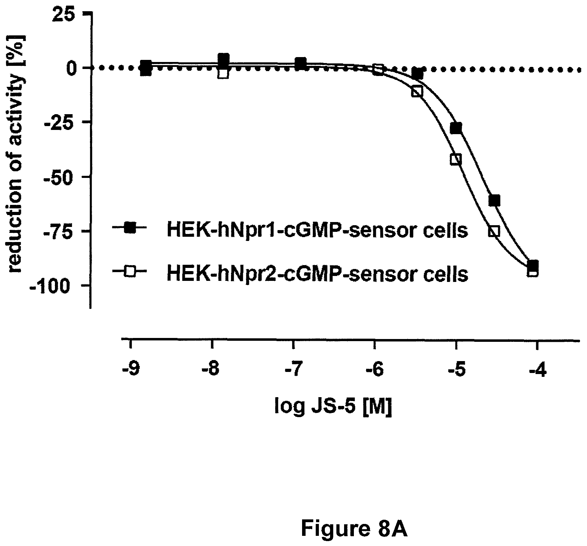

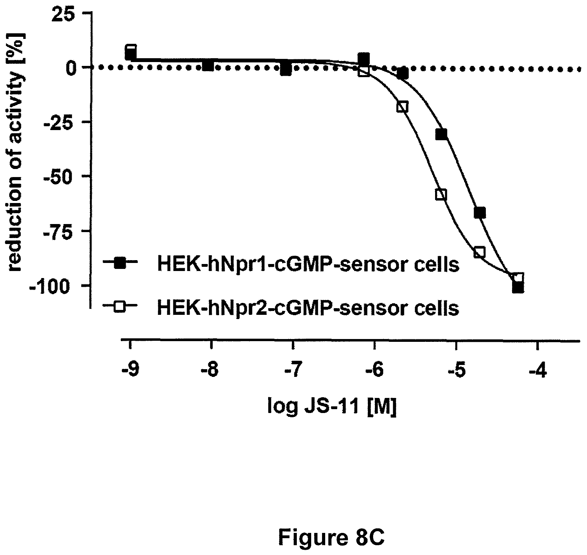

It was determined that three candidates, JS-5, JS-8, and JS-11, inhibit hNpr1 activity, but do not attenuate SNP dependent cGMP sensor activation, do not directly inhibit luciferase, and are not cytotoxic (Figures 3A-3F). However, these compounds also inhibited hNpr2 with similar potency to hNpr1 (Figures 8A-C), suggesting that the inhibitors identified do not distinguish between hNpr1 and hNpr2. The structures of JS-5, JS-8, and JS-11 are similar to each other (Figures 3B, 3D, and 3F), suggesting a common mode of action and a potential common binding site.

EXAMPLE 2 (comparative)

This example demonstrates that A-71915 is a partial agonist of Npr1.

It has been reported that the Npr1 antagonist A-71915 does not block acute itch responses elicited by the intradermal injection of pruritogens in mice, but the reason has been heretofore unexplained. One potential explanation for why A-71915 does not effectively block itch *in vivo* is that it may be a poor inhibitor. To investigate this possibility, a method to measure inhibition of mouse Npr1 (mNpr1) by A-71915 was developed, discussed below.

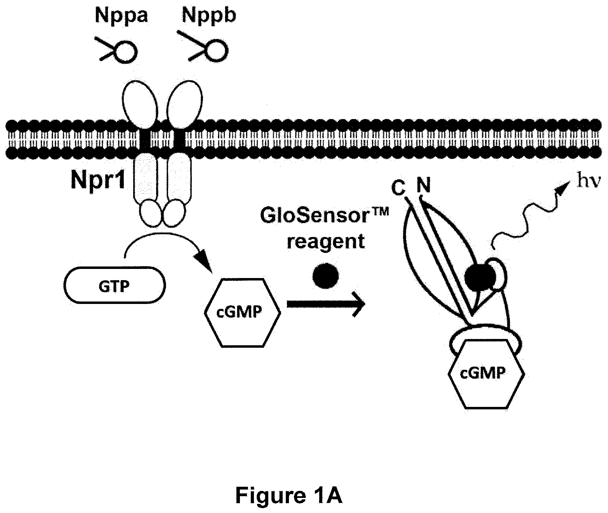

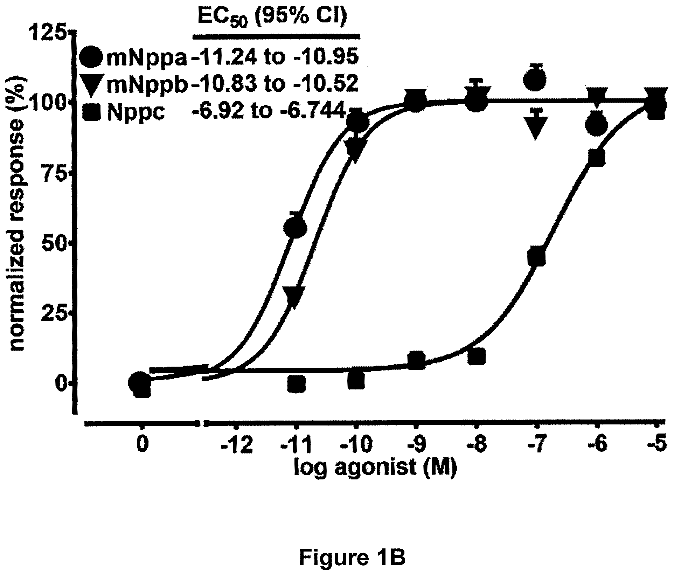

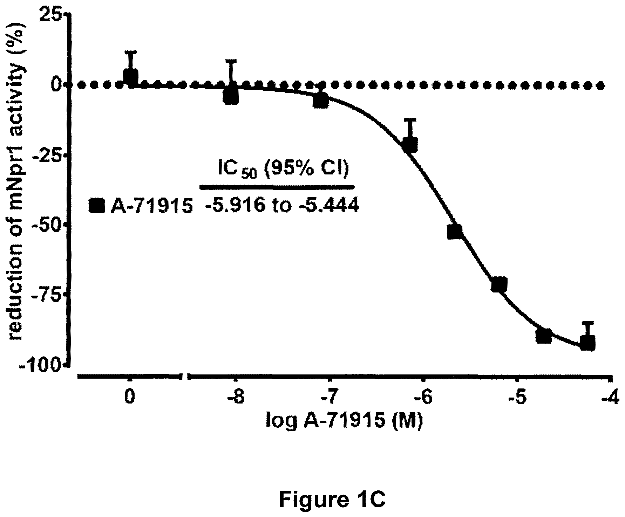

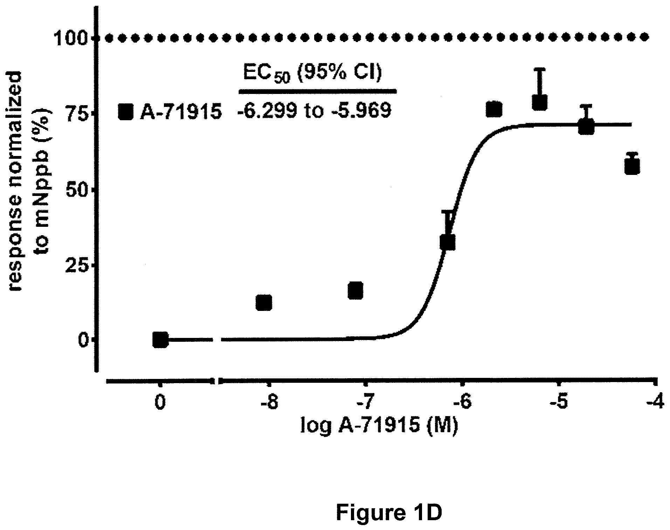

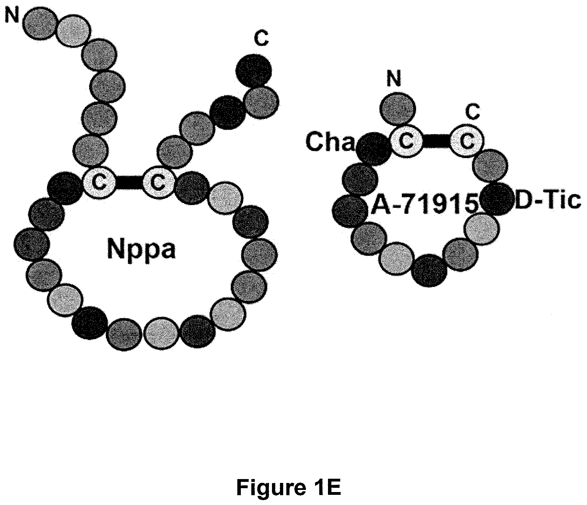

Because the Npr1 receptor is a ligand-dependent guanylate cyclase, measuring agonist-induced changes in intracellular cGMP levels was used to examine inhibition of Npr1. To measure the production of cGMP by Npr1, a circular permutated *Firefly* luciferase molecule which was functionally linked to the cGMP binding domain of human PDE5 (GloSensor™ technology pGS-40F) was used, as described in Example 1. This reporter molecule together with a luciferase substrate (GloSensor™ reagent) and a sensitive method for detection of luciferase activity (emitted light) permitted real-time measurement of cGMP levels in live cells (Figure 1A). The cGMP sensor was expressed in HEK293 cells and luciferase activity after stimulation with the NO-donor sodium nitroprusside (SNP), which activates ubiquitously expressed soluble guanylate cyclases, was examined. It was found that SNP generated a dose dependent increase in luciferase activity. In contrast, stimulation of these cGMP reporter cells with natriuretic peptides did not increase cGMP, indicating that HEK293 cells do not express detectable endogenous natriuretic peptide receptors. Next, mNpr1 and the cGMP sensor were transiently expressed in HEK293 cells and agonist dependent receptor activation was examined. It was found that natriuretic peptides dose dependently increased reporter activity and had stimulation potencies similar to those previously reported (Figure 1B). Next, the effects of A-71915 on mNpr1 were tested. It was found that A-71915 blocks agonist-induced mNpr1 activity with an $IC_{50}$ of 2 μM (Figure 1C). In the absence of natriuretic peptides, A-71915 evoked a dose-dependent activation of mNpr1, indicating that, instead of being a neutral antagonist, A-71915 acts as a partial agonist (Figure ID). It was hypothesized that because A-71915 is highly structurally related to natriuretic peptides, as shown in Figure 1E, it likely competes with natriuretic polypeptides for the same binding site. The fact that A-71915 is a partial agonist, not a full neutral antagonist, provides a potential explanation for the lack of efficacy of A-71915 in blocking itch behavior.

This example demonstrates that A-71915 can be used as a comparative compound to the candidate Npr1 antagonist compound provided herein, according to embodiments of the invention.

EXAMPLE 3

This example demonstrates the validation of the candidate hNpr1 inhibitor identified according to an embodiment of the invention.

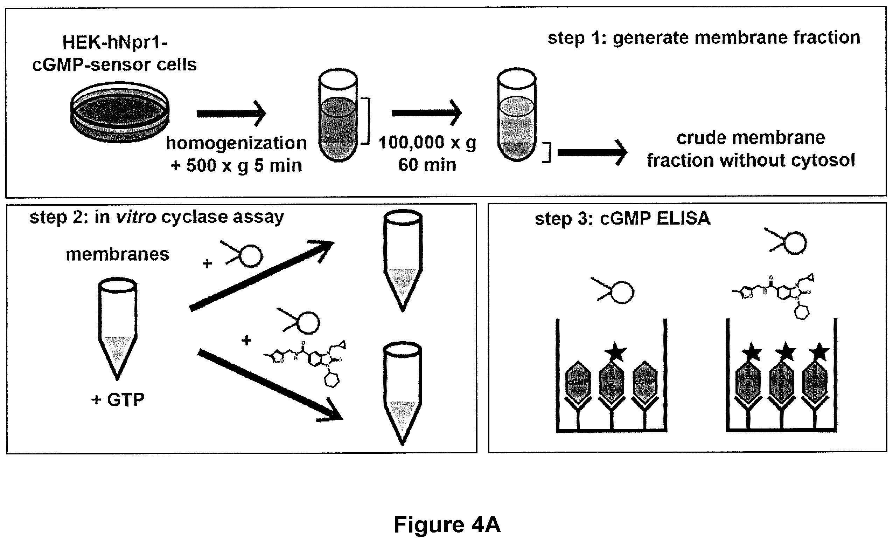

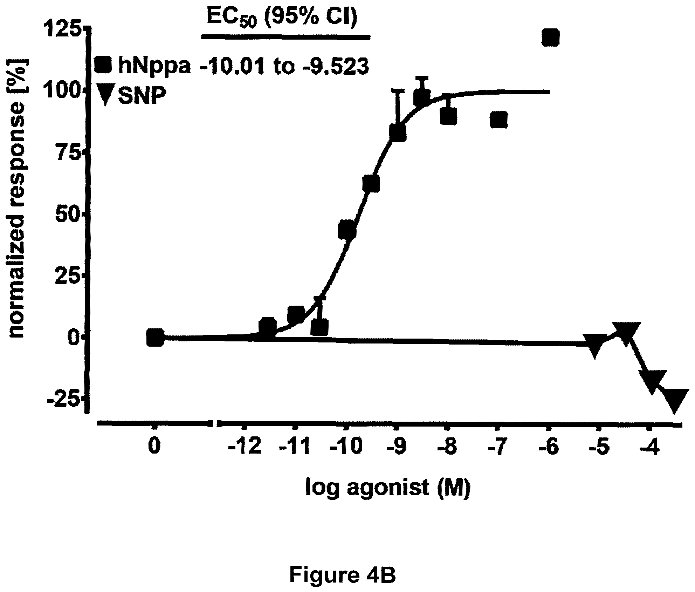

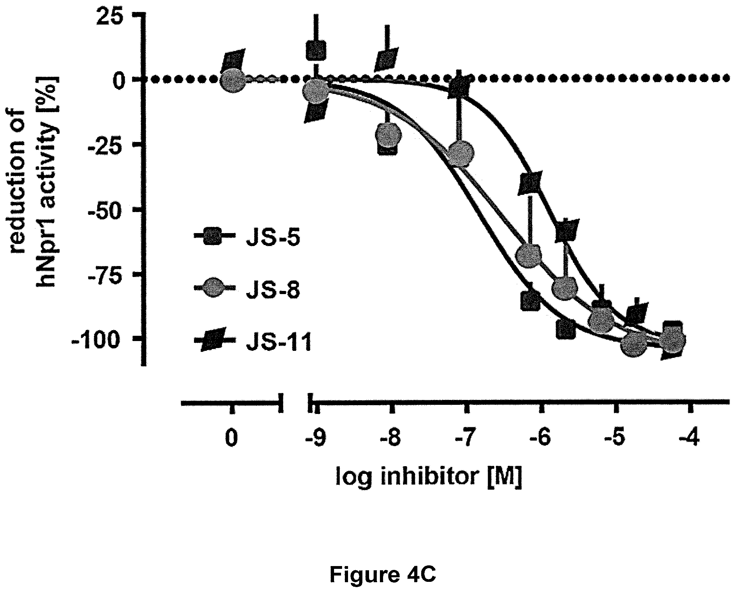

Twelve compounds were selected from those identified in Table 1 which could be obtained in large amounts at high purity (JS-3 through JS-14). Next, an independent strategy was developed to confirm the direct inhibition of hNpr1 by these compounds (Figure 4A). The approach used was to directly determine cGMP production by stimulated hNpr1 using an in *vitro* cyclase assay with subsequent measurement of cGMP using ELISA. The cGMP cyclase activity of membranes isolated from HEK293-hNpr1-cGMP sensor cells was measured. It was found that the activation of hNpr1 with hNppa increased cGMP levels, while activation of membranes with SNP did not (Figure 4B). All novel hNpr1 inhibitors could completely block cGMP production by hNpr1, as shown in Figure 4C and Table 4. In particular, membrane fractions were prepared from HEK-hNpr1-cGMP-sensor cells and in *vitro* cyclase assays were conducted to determine hNpr1 inhibition by the given compounds as described in Example 1. The inhibitory effects of Npr1 antagonists were quantified and the potency of this response was calculated by fitting the data to a four-parameter logistic curve. The results are set forth in Table 4.

**Table 4**. Cell-free Npr1 Membrane Cyclase Assay Confirmed That Candidate Compounds Are Specific hNpr1 Antagonists.

| Compound | $IC_{50}$ [µM] (mean ± SEM) | $I_{max}$ [%] (mean ±S EM) |
|---|---|---|
| JS-3* | 4.0 ± 1.2 | -98.2 ± 1.8 |
| JS-4* | 2.8 ± 0.04 | -96.1 ± 3.7 |
| JS-5* | 0.1 ± 0.1 | -99.8 ± 0.1 |
| JS-6* | 0.8 ± 1.9 | -99.4 ± 0.5 |
| JS-7* | 0.1 ± 0.3 | -98.4 ± 1.5 |
| JS-8* | 0.3 ± 0.4 | -98.0 ± 2.0 |
| JS-9* | 2.2 ± 1.8 | -95.3 ± 4.5 |
| JS-10* | 0.7 ± 0.6 | -95.2 ± 3.3 |
| JS-11** | 1.4 ± 0.1 | -99.7 ± 0.2 |
| JS-12* | 0.7 ± 0.1 | -99.0 ± 1.0 |
| JS-13* | 0.8 ± 0.9 | -94.4 ± 5.4 |
| JS-14* | 12.7 ± 4.9 | -99.0 ± 0.0 |
| A-71915*** | 0.1 ± 0.03 | -98.0 ± 3.5 |

* not claimed

** according to the claimed invention

*** comparative example

As noted, the reduction in hNpr1 activity of compounds JS-5, JS-8, and JS-11 is also shown in Figure 4C. The recorded potencies of inhibitory compounds on hNpr1 in the *in vitro* and cell-based assays, when corrected for differences in assay sensitivity, were very similar (Tables 5 and 6). In particular, data from tables 3, 4, and 6 (below) were used to calculate the inhibitor constant $K_i$ for novel Npr1 antagonists, using the Cheng-Prusoff equation

$$\left( K_i = \frac{IC_{50}}{1+\frac{[Npp]}{EC_{50}}} \right)$$

. This calculation corrects for differences in assay sensitivity thereby allowing the comparison of hNpr1 inhibition across different functional assays or the comparison of inhibition of Npr1 from different species.

**Table 5**. Calculated $K_i$ Values for hNpr1 and mNpr1 Are Similar Among Both Receptors and Different Assays.

| Compound | $K_i$ hNpr1 GloSensor™ [µM] (mean ± SEM) | $K_i$ hNpr1 cyclase assay [µM] (mean ± SEM) | $K_i$ mNpr1 GloSensor™ [µM] (mean ± SEM) |
|---|---|---|---|
| JS-3* | 0.50 ± 0.102 | 0.58 ± 0.179 | 0.25 ± 0.036 |
| JS-4* | 0.18 ± 0.002 | 0.41 ± 0.006 | 0.22 ± 0.070 |
| JS-5* | 1.18 ± 0.048 | 0.02 ± 0.020 | 0.42 ± 0.265 |
| JS-6* | 0.31 ± 0.023 | 0.11 ± 0.275 | 1.69 ± 1.395 |
| JS-7* | 0.19 ± 0.085 | 0.02 ± 0.044 | not determined |
| JS-8* | 0.20 ± 0.013 | 0.05 ± 0.064 | 0.10 ± 0.006 |
| JS-9* | 0.49 ± 0.215 | 0.32 ± 0.262 | 0.57 ± 0.0004 |
| JS-10* | 1.07 ± 1.079 | 0.11 ± 0.090 | 0.31 ± 0.037 |
| JS-11** | 0.30 ± 0.134 | 0.21 ± 0.008 | 0.10 ± 0.030 |
| JS-12* | 0.21 ± 0.0002 | 0.10 ± 0.015 | 0.26 ± 0.006 |
| JS-13* | 0.18 ± 0.011 | 0.12 ± 0.130 | 0.21 ± 0.046 |
| JS-14* | 0.50 ± 0.179 | 1.87 ± 0.723 | 0.32 ± 0.329 |
| A-71915*** | 0.18 ± 0.016 | 0.02 ± 0.004 | 0.05 ± 0.005 |

* not claimed

** according to the claimed invention

*** comparative example

For Table 6, HEK-293 cells transiently expressing mNpr1 and pGS-40F were stimulated with 1 nM mNppb 5 minutes after addition of candidate compounds or A-71915 and luminescence was measured for 30 minutes. Inhibitors were titrated to calculate apparent $IC_{50}$ values, as described in methods.

**Table 6**. Novel hNpr1 Inhibitors also Inhibit mNpr1.

| Compound | $IC_{50}$ [µM] (mean ± SEM) |
|---|---|
| JS-3* | 12 ± 1.8 |
| JS-4* | 11 ± 3.4 |

(continued)

| Compound | $IC_{50}$ [μM] (mean ± SEM) |
|---|---|
| JS-5* | 20 ± 12 |
| JS-6* | 82 ± 68 |
| JS-7* | n. d. |
| JS-8* | 4.7 ± 0.3 |
| JS-9* | 28 ± 0.02 |
| JS-10* | 15 ± 1.8 |
| JS-11** | 4.9 ± 1.5 |
| JS-12* | 13 ± 0.3 |
| JS-13* | 10 ± 2.2 |
| JS-14* | 15.8 ± 16 |
| A-71915*** | 2.2 ± 0.3 |
| * not claimed<br>** according to the claimed invention<br>*** comparative example | |

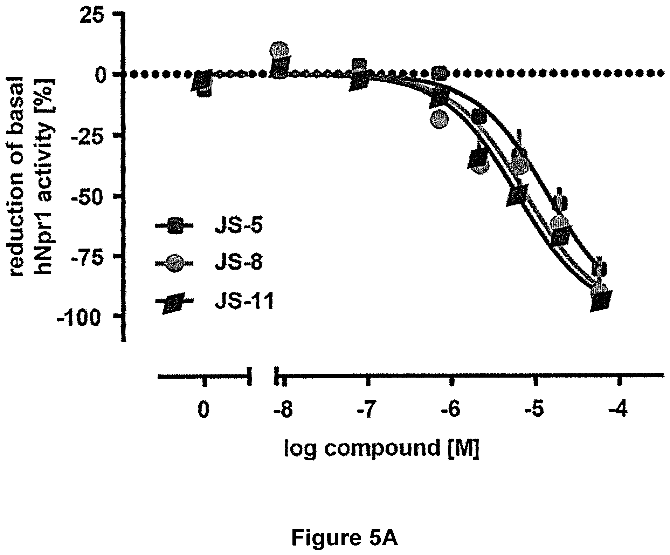

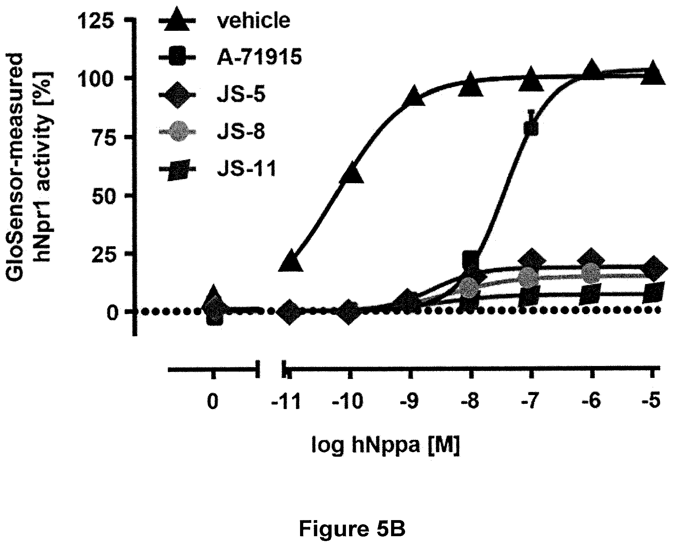

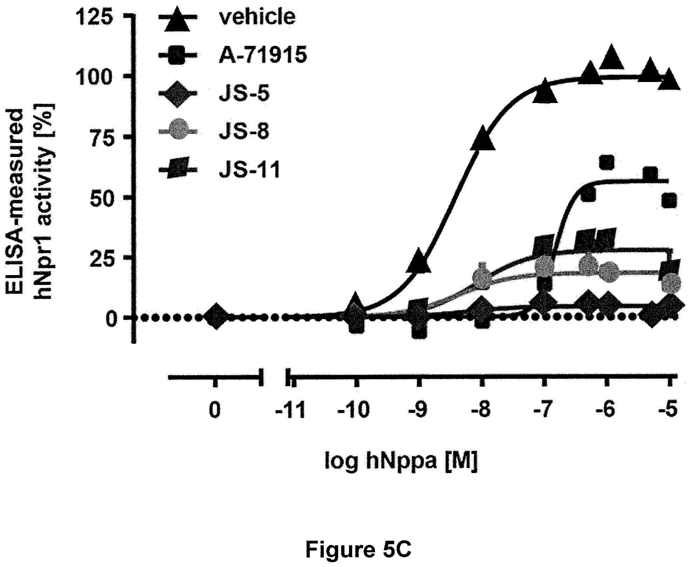

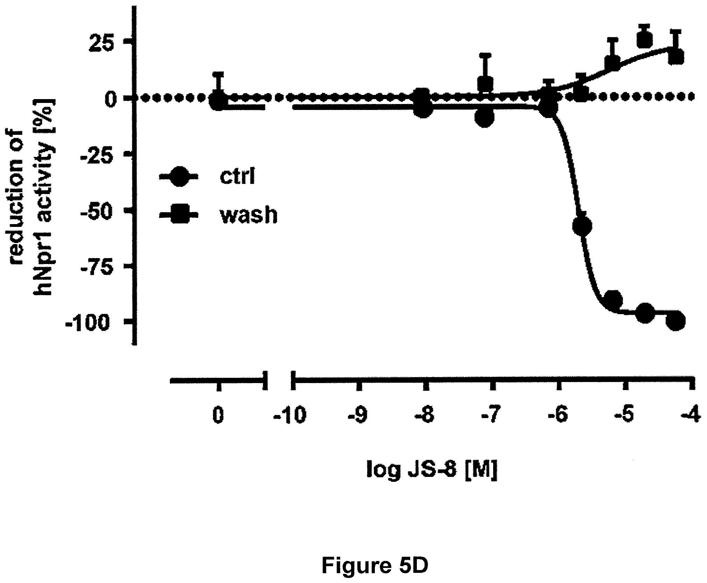

During the screening, it was found that both the basal and agonist-induced hNpr1 activity were inhibited by the JS-5, JS-8, and JS-11 inhibitors (Figure 5A). Because A-71915 behaves like a weak partial agonist, it was hypothesized that the compounds identified might inhibit hNpr1 via a different mechanism from A-71915. To explore this further, hNppa was titrated against fixed concentrations of A-71915 and the three antagonists JS-5, JS-8, JS-11 (Figures 5B-C). While A-71915 induced a right-shift in hNppA potency without any effect on maximal efficacy, JS-5, JS-8, and JS-11 reduced maximal responses even at extremely high hNppa concentrations ($10^5$-fold higher than $EC_{50}$). This lack of effect of increased agonist concentrations could either indicate a non-competitive inhibition of hNpr1 or could be explained by the slow dissociation of the antagonists from hNpr1. If the latter were true, it would be expected that washing Npr1 expressing cells after incubation with antagonist would have little effect on receptor inhibition. To examine this possibility, the HEK-hNpr1-cGMP-sensor cells were treated with JS-8 for 5 minutes and, after a 5-minute washing step, cells were stimulated with 60 pM hNppa. Inhibition of hNppa-induced hNpr1 activation after washing was compared to the control condition (ctrl; cells acutely pre-treated with JS-8 for 5 minutes and immediately stimulated with 60 pM hNppa). It was found that even a single washing step was sufficient to completely recover hNpr1 activity (Figure 5D), suggesting that the antagonists identified do not have slow dissociation, but likely block hNpr1 via a reversible non-competitive mechanism.

EXAMPLE 4

This example demonstrates the inhibition of itch in mouse models by the Npr1 antagonist compound identified according to an embodiment of the invention. To determine whether the identified inhibitors of Npr1 could alleviate itch, the inhibitory effect of the identified compounds (JS-3 through JS-14) on mNpr1 was examined. Comparison of inhibition efficacy on hNpr1 and mNpr1 revealed, when corrected for differences in assay sensitivity, that most of the identified compounds had similar potencies (Tables 5 and 6), suggesting they might block itch *in vivo* in a mouse model of itch.

As a proof-of-concept, the ability of one of the identified compounds to inhibit itch responses was investigated. Compound JS-11 (according to the claimed invention) was chosen because of its relatively high-water solubility, high membrane permeability (7.4 x $10^{-6}$ cm/sec), and moderate half-life (12.4 min). The behavioral measurements of mouse scratching behavior were assessed as follows.

*Behavioral Measurement of Acute Scratching Behavior*

All experiments using mice followed NIH guidelines and were approved by the National Institute of Dental and Craniofacial Research ACUC. Behavioral assessment of scratching behavior was conducted as follows: 6-8-week old female C57BL/6N mice (Envigo) were injected intraperitoneally with JS-11 (163 μg) or DMSO (20%) as a vehicle control. Ten minutes later, 100 μg histamine diluted in 10 μl PBS or 8.9 μg CYM5442 diluted in 10 μl $ddH_2O$ was injected

subcutaneously into the nape of the neck. Scratching behavior was recorded for 30 minutes and is presented in bouts per 30 minutes. One bout was defined as scratching behavior towards the injection site between lifting the hind leg from the ground and either putting it back on the ground or guarding the paw with the mouth. To control for motor impairment by JS-11, rota-rod performance was also assessed 10 minutes after intraperitoneal injection.

*Behavioral Measurement of Chronic Scratching Behavior*

The contact hypersensitivity model was performed as follows: the back of 6-8-week old female C57BL/6N mice (Envigo) was shaved with electric clippers. After 2 days, 25 μl of 0.5% (v/v) dinitro-fluoro-benzene (DNFB) diluted in a 4:1 mixture of acetone and olive oil was applied to the shaved back skin. Hapten challenge was performed 5 days after sensitization by applying 40 μl of 0.2% (v/v) DNFB in the same vehicle on the left ear and 40 μl of vehicle alone on the right ear. After 24 hours, baseline scratching was observed for 30 minutes. Mice were then injected intraperitoneally with JS-11 (163 μg) or DMSO (20%) as a vehicle control. After 10 minutes, scratching was again observed for 30 minutes. To assess skin inflammation, ear thickness was measured with a thickness gauge (Mitutoyo, Aurora, IL) before and 30 minutes after JS-11/DMSO injections and normalized to ear thickness before hapten challenge. Hapten challenge (left ear) induced significantly more skin inflammation than application of vehicle alone (right ear).

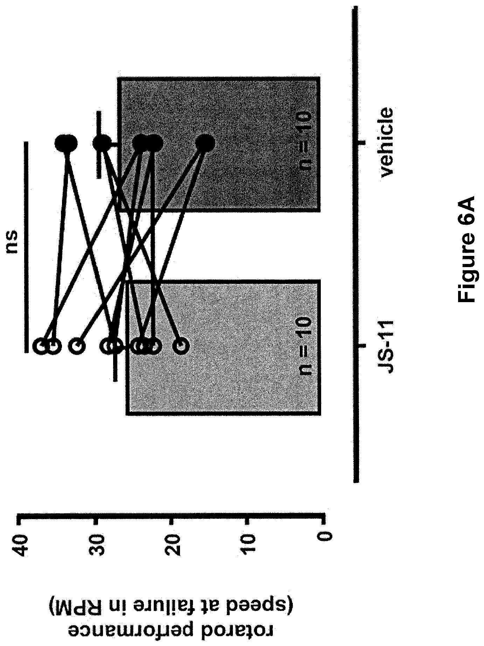

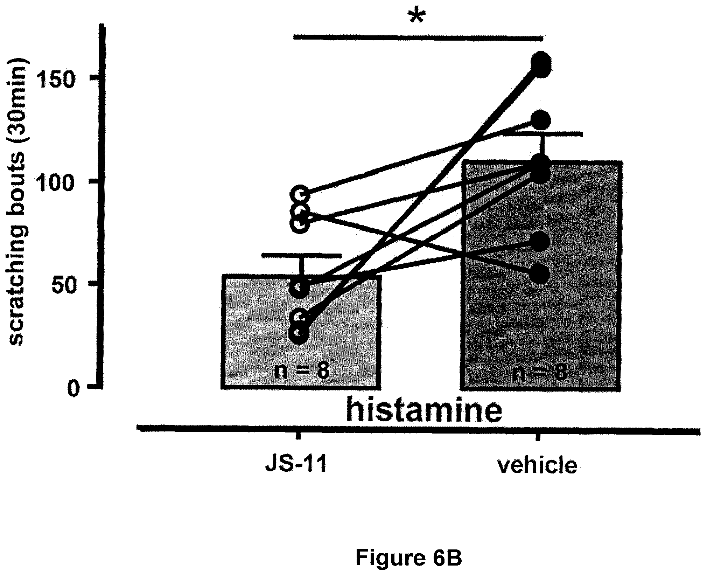

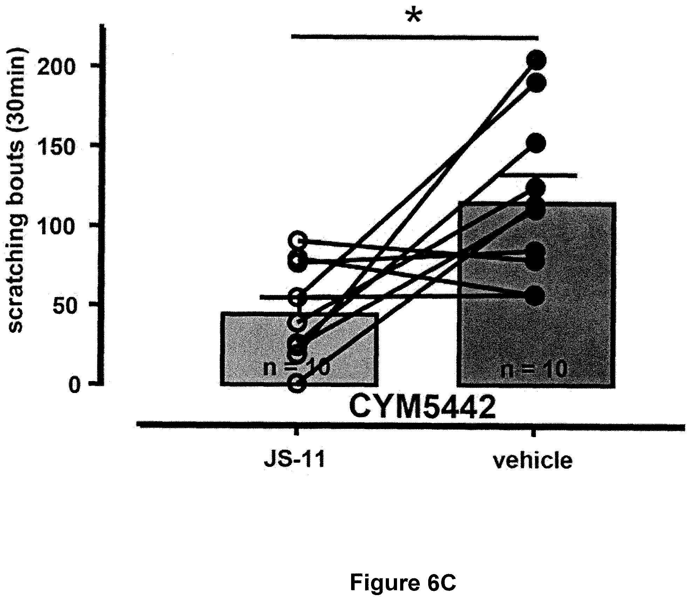

Intraperitoneal injection of 163 μg JS-11 produced no gross change in overall mouse behavior and rotarod measurements showed that JS-11 induced no impairment of motor coordination ten minutes after JS-11 treatment (Figure 6A). Next, whether scratching responses were attenuated by JS-11 was tested, as described in Example 5. Scratching responses to pruritogens injected intradermally into the nape of the neck (100 μg histamine and 8.9 μg CYM5442) were significantly attenuated compared to paired controls by administration of JS-11. JS-11 reduced scratching responses to histamine by more than half (Figure 6B). To corroborate this result, the pruritogen CYM5442 which activates the sphingosine-1-phosphate receptor 1, was tested in sensory neurons. Pairwise comparison showed that JS-11 strongly attenuated itch response (Figure 6C). Significant differences were assessed by using paired Student's t-test (histamine: p=0.0221, and CYM5442: p=0.0128). Data represent means ± SEM of n = 10 (Figures 6A and 6C) and 8 animals (Figure 6B).

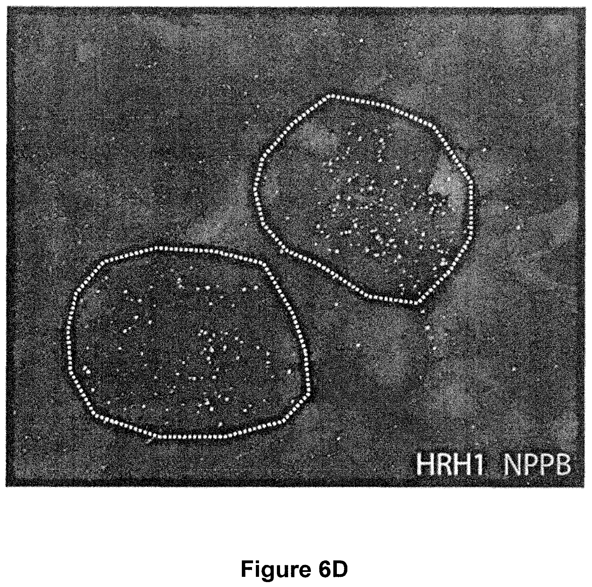

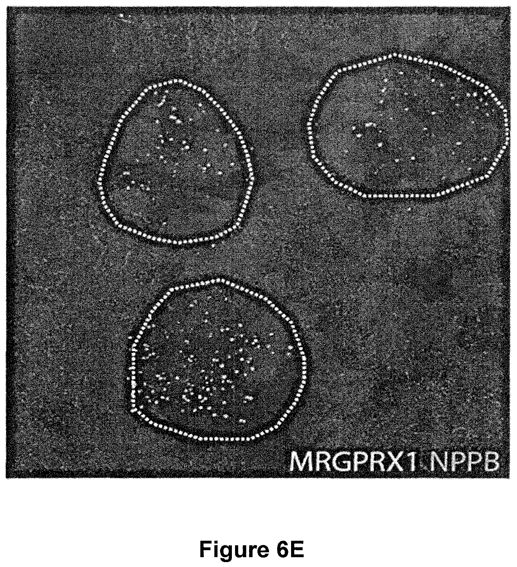

Next, to investigate whether Nppb is co-expressed with known itch receptors, double labelling ISH (in situ hybridization) on human DRG (dorsal root ganglion) sections with Nppb and the histamine receptor HRH1, and MRGPRX1, a receptor of the pruritic bovine adrenal medulla peptide 8-22 and the anti-malaria drug chloroquine was performed. It was found that HRH1 is exclusively co-expressed with Nppb (Figure 6D; 106 out of 107 DRG neurons) and that all MRGPRX1-positve cells co-express Nppb, although some Nppb expressing cells were MRGPRX1 negative (Figure 6E; 121 of 150 $NPPB^+$ DRG neurons express MRGPRX1).

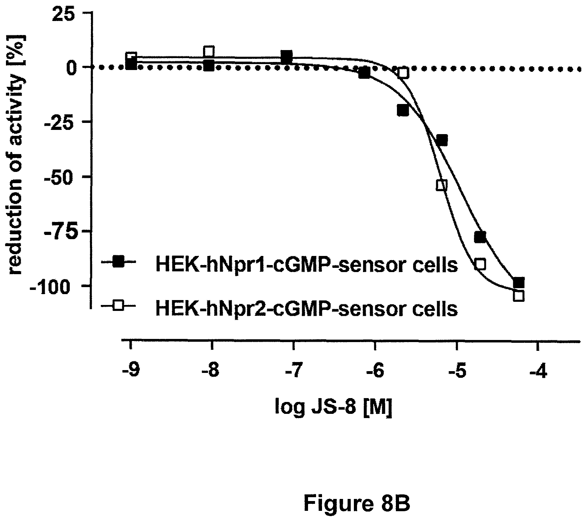

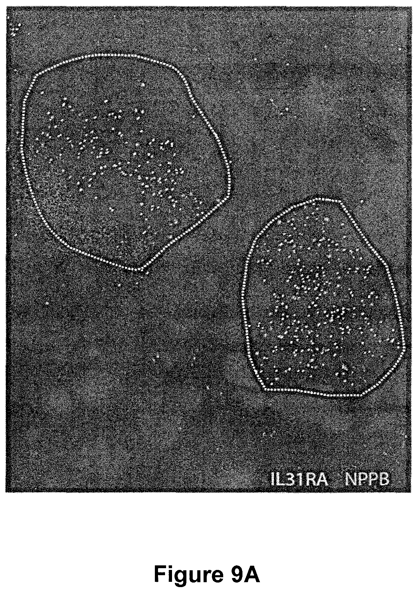

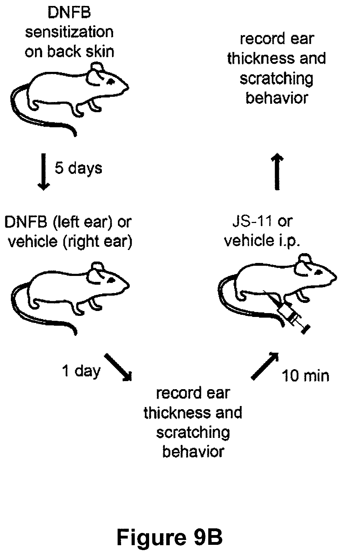

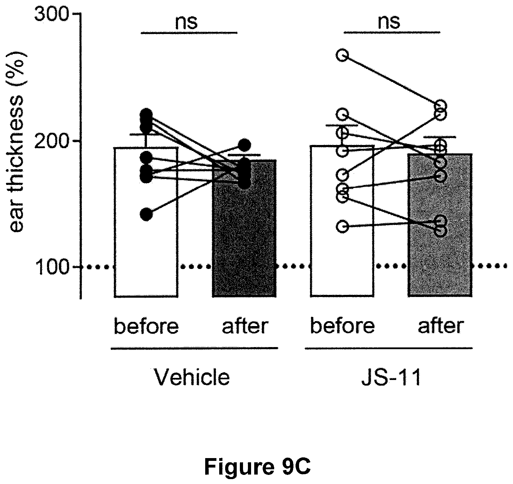

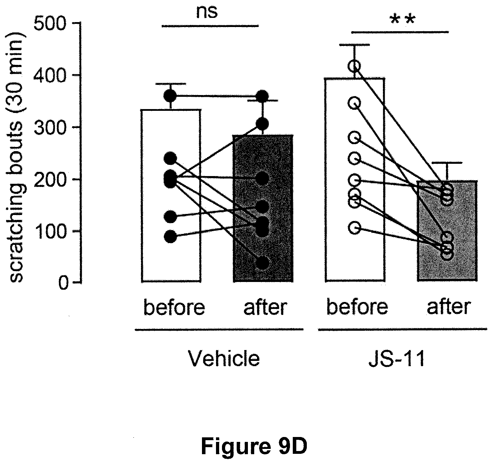

Next, whether Nppb is expressed with the receptor for interleukin-31 (IL-31), IL-31 receptor A (IL31RA) was investigated. As with HRH1 and MRGPRX1 itch receptors, ISH revealed that human DRG neurons expressing Nppb co-express IL31RA (Figure 9A; 119 of 126 DRG neurons). To further investigate the significance of this co-expression *in vivo,* the potential for blocking IL31-mediated itch with Npr1 antagonist in a mouse model of contact hypersensitivity was investigated (Figure 8B). In this model, scratching but not skin inflammation is dependent on IL-31. As assessed by measuring ear thickness after hapten challenge, acute blockade with JS-11 had no effect on skin inflammation (Figure 9C). By contrast, JS-11 attenuated hapten-induced scratching responses by about a half (Figure 9D).

This example demonstrates that inhibition of Npr1 by JS-11, and blocking Nppb neurotransmission are viable approaches for attenuation of human itch. These results also indicate that ongoing peripheral drive contributes to pruritus in a model of persistent inflammatory dermatitis and suggests that clinically, blocking Npr1 might be a valuable approach to treat patients with chronic itch.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description.

## Claims

1. A pharmaceutical composition comprising (a) a pharmaceutically acceptable carrier and (b) a molecular inhibitor of Natriuretic polypeptide receptor 1 ("Npr1 "), wherein the molecular inhibitor of Npr1 is 1-cyclohexyl-3-(cyclopropylmethyl)-N-((3-methylisoxazol-5-yl)methyl)-2-oxo-2,3-dihydro-1H-benzo[d]imidazole-5-carboxamide.

2. The pharmaceutical composition of claim 1, wherein the Npr1 is the human isoform of Npr1 ("hNpr1").

3. A pharmaceutical composition comprising (a) a pharmaceutically acceptable carrier and (b) a molecular inhibitor of Npr1, for use in the treatment, reduction, or prevention of acute and/or chronic pruritus in a mammal, wherein the molecular inhibitor of Npr1 is 1-cyclohexyl-3-(cyclopropylmethyl)-N-((3-methylisoxazol-5-yl)methyl)-2-oxo-2,3-dihydro-1H-benzo[d]imidazole-5-carboxamide.

4. The pharmaceutical composition for the use of claim 3, wherein the Npr1 is hNpr1.

5. The pharmaceutical composition for the use according to claim 3 or 4, wherein the acute and/or chronic pruritus is associated with a skin condition.

6. The pharmaceutical composition for the use according to claim 5, wherein the skin condition is a fungal skin infection, a *Trichomonas* skin infection, eczema, or psoriasis.

7. The pharmaceutical composition for the use according to any of claims 3-6, wherein the pruritus is induced by a pruritogen wherein the pruritogen is histamine, chloroquine, endothelin (ET-1), 2-methyl serotonin (5HT), SLIGRL-NH2 (PAR2), or compound 48/80 (48/80).

8. The pharmaceutical composition for the use according to any of claims 3-6, wherein the pruritus is mediated by interleukin (IL)-31.

9. The pharmaceutical composition for the use according to claim 3 or 4, wherein the acute and/or chronic pruritus is associated with a systemic condition.

10. The pharmaceutical composition for the use according to claim 9, wherein the systemic condition is renal failure, liver damage, liver disease, acquired immune deficiency syndrome (AIDS), polycythemia vera, diabetes, hyperthyroidism, or cancer.

11. The pharmaceutical composition for the use according to claim 9, wherein the systemic condition is cirrhosis.

12. The pharmaceutical composition for the use according to claim 3 or 4, wherein the pruritus is associated with kidney dialysis or chemotherapy.

13. The pharmaceutical composition for the use according to any of claims 3-12, wherein the mammal is a human.

14. The pharmaceutical composition for the use according to any of claims 3-12, wherein the mammal is a dog.

15. The pharmaceutical composition for the use according to any of claims 3-12, wherein the mammal is a cat.

## Patentansprüche

1. Eine pharmazeutische Zusammensetzung, umfassend (a) einen pharmazeutisch annehmbaren Träger und (b) einen molekularen Inhibitor des natriuretischen Polypeptidrezeptors 1 ("Npr1"), wobei der molekulare Inhibitor von Npr1 1-Cyclohexyl-3-(cyclopropylmethyl)-N-((3-methylisoxazol-5-yl)methyl)-2-oxo-2,3-dihydro-1H-benzo[d]imidazol-5-carboxamid ist.

2. Die pharmazeutische Zusammensetzung nach Anspruch 1, wobei das Npr1 die menschliche Isoform von Npr1 ("hNpr1") ist.

3. Eine pharmazeutische Zusammensetzung, umfassend (a) einen pharmazeutisch annehmbaren Träger und (b) einen molekularen Inhibitor von Npr1 zur Verwendung bei der Behandlung, Verminderung oder Vorbeugung von akutem und/oder chronischem Juckreiz bei einem Säugetier, wobei der molekulare Inhibitor von Npr1 1-Cyclohexyl-3-(cyclopropylmethyl)-N-((3-methylisoxazol-5-yl)methyl)-2-oxo-2,3-dihydro-1H-benzo[d]imidazol-5-carboxamid ist.

4. Die pharmazeutische Zusammensetzung für die Verwendung nach Anspruch 3, wobei das Npr1 hNpr1 ist.

5. Die pharmazeutische Zusammensetzung für die Verwendung nach Anspruch 3 oder 4, wobei der akute und/oder chronische Juckreiz mit einer Hauterkrankung verbunden ist.

6. Die pharmazeutische Zusammensetzung für die Verwendung nach Anspruch 5, wobei der Hauterkrankung eine Pilzinfektion der Haut, eine *Trichomonas*-Hautinfektion, ein Ekzem oder eine Psoriasis ist.

7. Die pharmazeutische Zusammensetzung für die Verwendung nach einem der Ansprüche 3-6, wobei der Pruritus durch ein Pruritogen induziert wird, wobei das Pruritogen Histamin, Chloroquin, Endothelin (ET-1), 2-Methylserotonin (5HT), SLIGRL-NH2 (PAR2) oder die Verbindung 48/80 (48/80) ist.

8. Die pharmazeutische Zusammensetzung für die Verwendung nach einem der Ansprüche 3-6, wobei der Juckreiz durch Interleukin (IL)-31 vermittelt wird.

9. Die pharmazeutische Zusammensetzung für die Verwendung nach Anspruch 3 oder 4, wobei der akute und/oder chronische Juckreiz mit einer systemischen Erkrankung verbunden ist.

10. Die pharmazeutische Zusammensetzung für die Verwendung nach Anspruch 9, wobei der systemische Erkrankung Nierenversagen, Leberschaden, Lebererkrankung, erworbenes Immunschwächesyndrom (AIDS), Polycythemia vera, Diabetes, Hyperthyreose oder Krebs ist.

11. Die pharmazeutische Zusammensetzung für die Verwendung nach Anspruch 9, wobei die systemische Erkrankung Zirrhose ist.

12. Die pharmazeutische Zusammensetzung für die Verwendung nach Anspruch 3 oder 4, wobei der Juckreiz mit einer Nierendialyse oder Chemotherapie verbunden ist.

13. Die pharmazeutische Zusammensetzung für die Verwendung nach einem der Ansprüche 3-12, wobei das Säugetier ein Mensch ist.

14. Die pharmazeutische Zusammensetzung für die Verwendung nach einem der Ansprüche 3-12, wobei das Säugetier ein Hund ist.

15. Die pharmazeutische Zusammensetzung für die Verwendung nach einem der Ansprüche 3-12, wobei das Säugetier eine Katze ist.

**Revendications**

1. Composition pharmaceutique comprenant (a) un support pharmaceutiquement acceptable et (b) un inhibiteur moléculaire de récepteur de polypeptide natriurétique 1 (« Npr1 »), sachant que l'inhibiteur moléculaire de Npr1 est du 1-cyclohexyl-3-(cyclopropylméthyl)-N-((3-méthylisoxazol-5-yl)méthyl)-2-oxo-2,3-dihydro-1H-benzo[d]imidazole-5-carboxamide.

2. La composition pharmaceutique de la revendication 1, sachant que le Npr1 est l'isoforme humaine de Npr1 (« hNpr1 »).

3. Composition pharmaceutique comprenant (a) un support pharmaceutiquement acceptable et (b) un inhibiteur moléculaire de Npr1, pour utilisation dans le traitement, la réduction, ou la prévention de prurit aigu et/ou chronique chez un mammifère, sachant que l'inhibiteur moléculaire de Npr1 est du 1-cyclohexyl-3-(cyclopropylméthyl)-N-((3-méthylisoxazol-5-yl)méthyl)-2-oxo-2,3-dihydro-1H-benzo[d]imidazole-5-carboxamide.

**4.** La composition pharmaceutique pour l'utilisation de la revendication 3, sachant que le Npr1 est hNpr1.

**5.** La composition pharmaceutique pour l'utilisation selon la revendication 3 ou 4, sachant que le prurit aigu et/ou chronique est associé à un état de la peau.

**6.** La composition pharmaceutique pour l'utilisation selon la revendication 5, sachant que l'état de peau est une infection mycosique de la peau, une infection par *Trichomonas* de la peau, un eczéma, ou un psoriasis.

**7.** La composition pharmaceutique pour l'utilisation selon l'une quelconque des revendications 3 à 6, sachant que le prurit est induit par un pruritogène, sachant que le pruritogène est de l'histamine, de la chloroquine, de l'endothéline (ET-1), de la 2-méthyl sérotonine (5HT), SLIGRL-NH2 (PAR2), ou un composé 48/80 (48/80).

**8.** La composition pharmaceutique pour l'utilisation selon l'une quelconque des revendications 3 à 6, sachant que le prurit est médié par de l'interleukine (IL)-31.

**9.** La composition pharmaceutique pour l'utilisation selon la revendication 3 ou 4, sachant que le prurit aigu et/ou chronique est associé à un état systémique.

**10.** La composition pharmaceutique pour l'utilisation selon la revendication 9, sachant que l'état systémique est une insuffisance rénale, une affection du foie, une maladie du foie, un syndrome d'immunodéficience acquise (SIDA), polycythemia vera, un diabète, un hyperthyroïdisme, ou un cancer.

**11.** La composition pharmaceutique pour l'utilisation selon la revendication 9, sachant que l'état systémique est une cirrhose.

**12.** La composition pharmaceutique pour l'utilisation selon la revendication 3 ou 4, sachant que le prurit est associé à une dialyse ou une chimiothérapie du rein.

**13.** La composition pharmaceutique pour l'utilisation selon l'une quelconque des revendications 3 à 12, sachant que le mammifère est un humain.

**14.** La composition pharmaceutique pour l'utilisation selon l'une quelconque des revendications 3 à 12, sachant que le mammifère est un chien.

**15.** La composition pharmaceutique pour l'utilisation selon l'une quelconque des revendications 3 à 12, sachant que le mammifère est un chat.

Nppa
Nppb
Npr1
GTP
cGMP
GloSensor™
reagent
C
N
cGMP
hv

Figure 1A

EC50 (95% CI)
mNppa -11.24 to -10.95
mNppb -10.83 to -10.52
Nppc -6.92 to -6.744
normalized response (%)
125
100
75
50
25
0
log agonist (M)
0
-12
-11
-10
-9
-8
-7
-6
-5

Figure 1B

reduction of mNpr1 activity (%)
25
0
-25
-50
-75
-100
0
-8
-7
-6
-5
-4
log A-71915 (M)
$IC_{50}$ (95% CI)
A-71915 -5.916 to -5.444

Figure 1C

EC50 (95% CI)
A-71915 -6.299 to -5.969
response normalized to mNppb (%)
100
75
50
25
0
log A-71915 (M)
-8
-7
-6
-5
-4


Figure 1D

N
C
C
C
Nppa
N
C
C
C
Cha
A-71915
D-Tic

Figure 1E

media
hNppa
hNppb
Nppc
injection
relative luminescence (%)
800
600
400
200
0
0
10
20
30
time (min)

Figure 2A

EC50 (95% CI)
hNppa -11.05 to -10.84
hNppb -10.17 to -9.921
Nppc -6.954 to -4.06
normalized response (%)
125
100
75
50
25
0
0
-12
-11
-10
-9
-8
-7
-6
-5
log agonist (M)

Figure 2B

△ media
■ hNppa
□ hNppb
● Nppc
◆ SNP
injection
relative luminescence (%)
1000
800
600
400
200
0
0
10
20
30
time (min)


Figure 2C

cGMP-reporter cells
EC50 (95% CI)
hNppa
SNP -4.409 to -4.19
Nppc
hNppb
normalized response (%)
100
75
50
25
0
0
-9
-8
-7
-6
-5
-4
log agonist (M)

Figure 2D

seed cells
incubate 24 h
add substrate
incubate 2 h
add compound
incubate 20 min
read
add agonist
incubate 30 min
read

Figure 2E

% activity
0
-50
-100
-8
-7
-6
-5
-4
log [compound], M
10,000
20,000
30,000
40,000
50,000
60,000
70,000
80,000
compound no.

Figure 2F

reduction of activity [%]
25
0
-25
-50
-75
-100
FLuc in vitro
cytoxicity
HEK-cGMP-sensor cells
HEK-hNpr1-cGMP-sensor cells
-9
-8
-7
-6
-5
-4
log JS-5 [M]
N–O
N
N

Figure 3A

Figure 3B

reduction of activity [%]
25
0
-25
-50
-75
-100
FLuc in *vitro*
cytoxicity
HEK-cGMP-sensor cells
HEK-hNpr1-cGMP-sensor cells
-9
-8
-7
-6
-5
-4
log JS-8 [M]
N–N
N
O
O
O
O

Figure 3C

Figure 3D

reduction of activity [%]
25
0
-25
-50
-75
-100
FLuc in vitro
cytoxicity
HEK-cGMP-sensor cells
HEK-hNpr1-cGMP-sensor cells
-9
-8
-7
-6
-5
-4
log JS-11 [M]
O
N
N
O
N
H
O-N

Figure 3E

Figure 3F

step 1: generate membrane fraction
HEK-hNpr1-
cGMP-sensor cells
homogenization
+ 500 x g 5 min
100,000 x g
60 min
crude membrane
fraction without cytosol
step 2: in vitro cyclase assay
membranes
+ GTP
+
+
step 3: cGMP ELISA
cGMP
conjugate

Figure 4A

hNppa
SNP
EC50 (95% CI)
hNppa -10.01 to -9.523
normalized response [%]
125
100
75
50
25
0
-25
0
-12
-11
-10
-9
-8
-7
-6
-5
-4
log agonist (M)

Figure 4B

reduction of
hNpr1 activity [%]
25
0
-25
-50
-75
-100
JS-5
JS-8
JS-11
0
-9
-8
-7
-6
-5
-4
log inhibitor [M]

Figure 4C

reduction of basal hNpr1 activity [%]
25
0
-25
-50
-75
-100
JS-5
JS-8
JS-11
0
-8
-7
-6
-5
-4
log compound [M]

Figure 5A

vehicle
A-71915
JS-5
JS-8
JS-11
GloSensor-measured hNpr1 activity [%]
125
100
75
50
25
0
0
-11
-10
-9
-8
-7
-6
-5
log hNppa [M]

Figure 5B

ELISA-measured hNpr1 activity [%]
125
100
75
50
25
0
vehicle
A-71915
JS-5
JS-8
JS-11
0
-11
-10
-9
-8
-7
-6
-5
log hNppa [M]

Figure 5C

reduction of
hNpr1 activity [%]
25
0
-25
-50
-75
-100
ctrl
wash
0
-10
-9
-8
-7
-6
-5
-4
log JS-8 [M]

Figure 5D

rotarod performance
(speed at failure in RPM)
40
30
20
10
0
ns
n = 10
n = 10
JS-11
vehicle

Figure 6A

*
scratching bouts (30min)
150
100
50
0
n = 8
n = 8
histamine
JS-11
vehicle

Figure 6B

scratching bouts (30min)
200
150
100
50
0
*
n = 10
n = 10
JS-11
vehicle
CYM5442

Figure 6C

HRH1 NPPB

Figure 6D

MRGPRX1 NPPB

Figure 6E

relative luminescence (%)
800
600
400
200
0
media
Nppc
injection
0
10
20
30
time (min)

Figure 7A

hNppa
hNppb
Nppc
$EC_{50}$ (95% CI)
-6.795 to -6.222
-6.406 to -6.103
-9.328 to -8.967
normalized response (%)
125
100
75
50
25
0
0
-12
-11
-10
-9
-8
-7
-6
-5
log agonist (M)


Figure 7B

reduction of activity [%]
25
0
-25
-50
-75
-100
HEK-hNpr1-cGMP-sensor cells
HEK-hNpr2-cGMP-sensor cells
-9
-8
-7
-6
-5
-4
log JS-5 [M]

Figure 8A

reduction of activity [%]
25
0
-25
-50
-75
-100
HEK-hNpr1-cGMP-sensor cells
HEK-hNpr2-cGMP-sensor cells
-9
-8
-7
-6
-5
-4
log JS-8 [M]

Figure 8B

reduction of activity [%]
25
0
-25
-50
-75
-100
HEK-hNpr1-cGMP-sensor cells
HEK-hNpr2-cGMP-sensor cells
-9
-8
-7
-6
-5
-4
log JS-11 [M]

Figure 8C

IL31RA NPPB

Figure 9A

DNFB
sensitization
on back skin
5 days
DNFB (left ear) or
vehicle (right ear)
1 day
record ear
thickness and
scratching
behavior
10 min
JS-11 or
vehicle i.p.
record ear
thickness and
scratching
behavior

Figure 9B

ear thickness (%)
300
200
100
ns
ns
before
after
before
after
Vehicle
JS-11

Figure 9C

scratching bouts (30 min)
500
400
300
200
100
0
ns
**
before
after
before
after
Vehicle
JS-11

Figure 9D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 000721
- US 039982
- US 20170014486 A1


### Non-patent literature cited in the description


- **MALO et al.** *Nat. Biotechnol.,* 2006, vol. 24, 167-175
- **INGLESE et al.** *PNAS USA,* 2006, vol. 103, 11473-11478
- **AUSTIN et al.** *Science,* 2004, vol. 306, 1138-1139
- **MISHRA et al.** *J. Neurosci.,* 2012, vol. 32, 8686-8695
- **JANG et al.** Identification of drug modulators targeting gene-dosage disease CMT1A. *ACS Chem Biol,* 2012, vol. 7, 1205-1213
- **JANG et al.** *ACS Chem. Bio.,* 2012, vol. 7, 1205-1213
- **AVDEEF et al.** Drug absorption in vitro model: filter-immobilized artificial membranes. 2. Studies of the permeability properties of lactones in Piper methysticum Forst. *Eur J Pharm Sci,* 2001, vol. 14, 271-280